(19) Europäisches Patentamt

European Patent Office

Office européen des brevets

(11) **EP 2 125 076 B1**

(12) **EUROPEAN PATENT SPECIFICATION**

(45) Date of publication and mention
of the grant of the patent:
**20.06.2012 Bulletin 2012/25**

(51) Int Cl.:
**A61M 5/142** *(2006.01)*        **A61F 5/00** *(2006.01)*
**A61K 9/00** *(2006.01)*

(21) Application number: **08710170.5**

(86) International application number:
**PCT/IL2008/000170**

(22) Date of filing: **07.02.2008**

(87) International publication number:
**WO 2008/104968 (04.09.2008 Gazette 2008/36)**

(54) **SPRAY ADMINISTRATION OF COMPOSITIONS INCLUDING ACTIVE AGENTS SUCH AS PEPTIDES TO THE GASTROINTESTINAL TRACT**

SPRÜHVERABREICHUNG VON ZUSAMMENSETZUNGEN MIT WIRKSTOFFEN, WIE Z. B. PEPTIDEN, IN DEN MAGEN-DARM-TRAKT

ADMINISTRATION PAR PULVÉRISATION DE COMPOSITIONS COMPRENANT DES AGENTS ACTIFS TELS QUE DES PEPTIDES AU TRACTUS GASTRO-INTESTINAL

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **26.02.2007 US 903289 P**

(43) Date of publication of application:
**02.12.2009 Bulletin 2009/49**

(73) Proprietor: **Duocure, Inc.**
**47220 Ramat-HaSharon (IL)**

(72) Inventor: **MAGAL, Elad**
**47220 Ramat-HaSharon (IL)**

(74) Representative: **Harrison IP Limited**
**1st Floor, Box Tree House**
**Northminster Business Park**
**Northfield Lane**
**York,**
**YO26 6QU (GB)**

(56) References cited:
WO-A-01/34088                WO-A-2006/035446
US-A1- 2004 172 142        US-A1- 2006 178 691
US-B1- 6 217 886

EP 2 125 076 B1

**Description**

FIELD AND BACKGROUND OF THE INVENTION

The present invention relates to administration of active agents such as active pharmaceutical ingredients and more particularly to the administration of active agents by spraying a sprayable composition including the active agent at a portion of the wall of the lumen of the gastrointestinal tract.

In the medical and health care arts there is a continuous search for methods of effective administration of active agents, whether by different routes (e.g., topical, oral, transdermal, subcutaneous, intravenous) or different types of compositions (e.g. liquids, gels, tablets, foams). One logical method used for thousands of years is through the gastrointestinal tract: a composition containing the active agent is orally ingested and passes through the gastrointestinal tract, during which passage the active agent acts, for example topically by treating a pathology on the lumen of the gastrointestinal tract or by stimulating sites such as chemoreceptors on the lumen of the gastrointestinal tract or for example, systemically by absorption through the walls of the gastrointestinal tract to enter the circulatory system.

Administration of active agents through the gastrointestinal tract is inefficient due to many factors including the varying chemical and physical environmental conditions along the gastrointestinal tract, the fact that most active agents act or are absorbed only at specific locations of the gastrointestinal tract and the fact that many active agents are susceptible to degradation of one or more organs of the gastrointestinal tract. As a result, efforts have been made to provide compositions that deliver an effective dose of an active agent to a specific location of the gastrointestinal tract. Such compositions include delayed-release compositions which release an active agent a predetermined time after ingestion or targeted-release compositions which release an active agent only upon exposure to conditions present in a specific location of the gastrointestinal tract.

An alternative approach is taught in PCT patent application PCT/IL20051001053 published as WO 2006/035446 of the Applicant in the context of treating conditions relating to overeating such as obesity. A central concept taught therein is the delivery of a beneficial stimulus at the "right time" (only when needed) at the "right place" (to a localized part of the body where most effective) which allows the "right dose" to be administered (no over- or under-dosing). Such an approach has the potential of increased efficacy, with less stimulation, fewer side effects and reduced chance for sensitization.

The specific teachings of WO 2006/035446 are based on the fact that the desire to eat is, in a large part, driven by hunger which stops upon the perception of satiation. Various mechanoreceptors and chemoreceptors in the gastrointestinal tract detect the degree of distension of the gastrointestinal tract and release satiety factors. Known satiety factors that are released to control food ingestion include Cholecystokinin (CCK), Bombesin, Gastrin-releasing peptide (GRP), Glucagon, Glucagon-like peptide (GLP-1), Enterostatin and Ghrelin. As there is a delay between the time of food ingestion and the release of the satiety factors, it is common for a person to overeat. Apart from the direct weight gain caused by consuming too much food, overeating also causes the base volume of the stomach to increase and the gastric mechanoreceptors to become insensitive to small increases of stomach volume. Thus, a positive-feedback loop with negative consequences is generated where a person inherently overeats as indication of satiety occurs only after satiety is reached, so that the person overeats, reducing the sensitivity of the satiety sensors, so that the indication of satiety is delayed even further.

To resolve this problem, WO 2006/035446 teaches a device capable of sensing a physiological change associated with food ingestion or hunger (i.e., identifying the "right time") and a mechanism adapted for directly stimulating a region of the body responsive to a gastrointestinal satiety agent which is implanted in the body (i.e., the "right place"). Specific mechanisms for sensing a physiological change associated with food ingestion or hunger include muscle activity sensors and pressure sensors. Specific mechanisms adapted for stimulation include drug dispensers, space-filling balloons, vagal-mechanoreceptor stimulating balloons and nerve-stimulating electrodes. Upon detection of a physiological change associated with food ingestion or hunger, the stimulation mechanism is activated giving the person a feeling of satiety. By detecting that a person is about to eat or has started eating before having consumed too much food and then stimulating a feeling of satiety, the teachings of WO 2006/035446 treat or control over-eating and related disorders such as obesity.

Further, WO 2006/035446 teaches the stimulation of satiety by administration of an active agent specifically to a region where that active agent is preferentially active, for example, the duodenum, the antral sphincter and the gastrointestinal wall where chemoreceptors sensitive to the active agent are found. Suggested active agents include usually orally administered anti-obesity drugs such as lipase inhibitors, CCK, CCK analogs, GLP-1, PYY, beta-3-adrenergic agonists such as CL 316243 (White CL., et al., 2004, Physiol. Behav. 82(2-3): 489-96), antagonists to the cannabinoid receptor (Lichtman AH and Cravatt BF, 2005, J. Clin. Invest. 115: 1130-3) and fat derived weight maintaining drugs such as leptin.

WO 2006/035446 teaches a variety of methods for stimulating a region responsive to a gastrointestinal satiety, including administration of an active agent by irrigation, injection or dispersion through a dispersion tube. Although novel and effective, the teachings of WO 2006/035446 do not specifically teach an optimum method of stimulating the feeling of satiety.

It would be highly advantageous to have an efficient method and a device for efficiently preventing and/or treating obesity incorporating at least some of the teachings of WO 2006/035446.

More generally, it would be highly advantageous to have a method and a device for administering active agents such as active pharmaceutical ingredients that have advantages over the methods known in the art.

US-B1-6217886 and WO 01/34088 disclose sprayable compositions comprising e.g. vasoactive intestinal peptides.

## SUMMARY OF THE INVENTION

The present invention successfully addresses at least some of the shortcomings of the prior art by providing a device according to claim 1 for administering sprayable pharmaceutical compositions including active agents such as active pharmaceutical ingredients (APIs) as a spray at a location in the gastrointestinal tract, the location generally selected as being preferred, ideal or in some way advantageous for administration of the active agent as well as, sprayable compositions according to claim 11. In embodiments the active ingredient is administered at a selected moment, the moment selected as being preferred, ideal or advantageous in some way for administration of the active agent. In embodiments, administration is of active ingredients that stimulate a perception of satiety upon detection of a physiological change associated with food ingestion or hunger.

The present invention is based, at least in part, on the discovery that spray administration of a sprayable pharmaceutical composition (in some embodiments a fluid composition, in some embodiments a liquid composition, especially a non-viscous liquid composition) containing an active agent (e.g., a peptide such as a peptide hormone, analogue or derivative, especially a peptide having no more than 40 amino residues, no more than 30 amino acid residues and even no more than 20 amino acid residues) at a specific location of the gastrointestinal tract, especially at selected moments, is an unexpectedly effective manner of administering the active agent.

There is presented a method of administering an active agent, comprising: a) deploying a sprayer of a device for administering a sprayable pharmaceutical composition in a specific location of a gastrointestinal tract of a subject (in embodiments a human, in embodiments a non-human animal) suffering from a condition, preferably so that the sprayer is substantially fixed in place; b) providing a sprayable pharmaceutical composition comprising an active agent and a pharmaceutically acceptable carrier, the active agent effective in treating the condition; and c) when necessary, spraying a dose of the sprayable composition through the sprayer against a portion of a luminal wall of the gastrointestinal tract of the subject thereby administering the active agent so as to treat the condition.

In some examples the method is a topical method of administration, the active agent treating the condition, for example by interacting with chemoreceptors apparent on or in the gastrointestinal tract luminal wall.

In some cases, the method is a systemic method of administration, the active agent treating the condition, for example, by being absorbed by the gastrointestinal tract so as to treat the condition.

In some examples, deploying the sprayer is such that the dose of spray is directed towards the luminal wall of the gastrointestinal tract.

In some examples deploying the sprayer comprises anchoring the sprayer at the specific location, preferably substantially tensionless anchoring.

The active agent is a peptide, such as a peptide hormone, an analogue thereof or a derivative thereof. In some embodiments the active agent is a peptide having no more than 40 amino residues, no more than 30 amino acid residues and even no more than 20 amino acid residues.

In some cases the specific location of the gastrointestinal tract is selected from the group consisting of esophagus, stomach, antrum, antral sphincter, fundus, pylorus, small intestine, duodenum, jejunum, ileum, large intestine, caecum, vermiform appendix, colon, ascending colon, transverse colon, descending colon, sigmoid flexure and rectum. For example, in some embodiments, a peptide hormone (or an analogue thereof or a derivative thereof) is sprayed at the luminal wall of a duodenum (in embodiments, the superior portion of the duodenum) in order to interact with chemoreceptors apparent thereupon.

The method may further comprise functionally associating a reservoir holding the sprayable composition with the sprayer. In some embodiments, functionally associating the reservoir with the sprayer comprises deploying the reservoir in the gastrointestinal tract, for example, in the stomach. In some cases, functionally associating the reservoir with the sprayer comprises implanting the reservoir in the body, for example subcutaneously or in the abdomen.

Typical conditions from which the subject suffers for which the method presented is useful include, but are not limited to obesity, bulimia, eating disorders, overeating, diabetes-related obesity, metabolic syndrome, inflammatory bowel disease, infections such as of helicobacter pylori, cardiovascular pathologies such as angina or arrhythmia, asthma and allergies.

In some examples the necessity of administering the dose of the active agent is periodic, that is, doses are administered periodically according to a schedule, for example when the method of the present invention is used to provide maintenance doses of an active agent for treating a chronic condition.

In some cases, the necessity of administering the dose of the active agent is determined by detection of an event of significance for administration of the active agent and administration is initiated a specified time subsequent to detection of the event.

In some embodiments the detection of the event is non-automatic, for example is performed by a care-giver, medical professional or the subject self.

In some embodiments, the detection of the event is automatically performed with the help of an event detector functionally associated with the sprayer.

In some embodiments, the event is a physiological change, such as gastrointestinal activity indicative of an event such as food ingestion and hunger. In such embodiments, a typical sprayable composition comprises, as an active agent, a gastrointestinal satiety agent or anti-food absorption drug such as a lipase inhibitor

In some embodiments, the event comprises angina or arrhythmia, for example in a subject suffering from a cardiovascular pathology. In such an embodiment a typical active agent is an anti-anginal or anti-arrhythmic such as adenosine or nitrates (e.g., isosorbide dinitrate, isosorbide mononitrate or nitroglycerin) administered in the ileum.

In some embodiments, the event comprises an asthmatic attack, for example in a subject suffering from asthma. In such an embodiment a typical active agent is an anti-asthma agent such as a systemic bronchial activator (e.g., salbutamol) administered in the ileum.

In some embodiments, the event comprises an allergic reaction, especially a systemic allergic reaction, for example in a subject suffering from allergic reaction as a result of a bee sting or the like. In such an embodiment a typical active agent is an anti allergy agent (e.g., epinephrine) administered in the ileum.

In some cases, functionally associating a reservoir with the sprayer comprises deploying a reservoir in the gastrointestinal tract, especially in the stomach, and charging the reservoir with the sprayable composition. In some cases, the reservoir is periodically recharged with a sprayable composition while deployed in the gastrointestinal tract.

In some examples, functionally associating a reservoir with the sprayer comprises implanting a reservoir in the body of the subject, especially subcutaneously, and charging the reservoir with the sprayable composition. In some examples, the reservoir is periodically recharged with a sprayable composition while implanted.

A method of administering an active agent may comprise: a) deploying a sprayer of a device of in a specific location of a gastrointestinal tract (e.g., the duodenum such as the superior portion of the duodenum) of a subject suffering from a condition; b) providing a sprayable pharmaceutical composition comprising a peptide active agent and a pharmaceutically acceptable carrier, the active agent effective in treating the condition; and c) when necessary, dispensing a dose of the sprayable composition through the sprayer so that the active agent interacts with chemoreceptors apparent on the luminal wall of the gastrointestinal tract of the subject thereby administering the active agent so as to treat the condition wherein the active agent is a peptide hormone, an analogue thereof or a derivative thereof. In some cases, the condition from which the subject suffers is a condition selected from the group comprising obesity, bulimia, eating disorders, overeating, diabetes-related obesity and metabolic syndrome. In some examples, the active agent is a gastrointestinal satiety agent. In some examples, the active agent is a CCK analogue or CCK derivative, or a CCK receptor agonist such as CCK-8. In some examples, the necessity of administering the dose of the active agent is determined by detection of an event of significance for administration of the active agent. In some examples, the event is a physiological change such as gastrointestinal tract activity, food ingestion or hunger. In some examples the peptide active agent has no more than 40 amino residues, no more than 30 amino acid residues and even no more than 20 amino acid residues.

According to the present invention there is provided a device for administering a sprayable pharmaceutical composition including an active agent to a luminal wall of the gastrointestinal tract of a subject, comprising: a) a sprayer configured for deployment (preferably substantially fixed deployment) in a gastrointestinal tract; b) a pressure generator configured to dispense a dose of a sprayable composition out through the sprayer as a spray upon actuation; and c) an actuator for actuating the pressure generator upon being triggered.

According to some embodiments of the present invention there is also provided a device useful for administering a sprayable composition to a luminal wall of the duodenum of a subject, comprising: a) a sprayer configured for deployment in a duodenum; b) a feeder tube including a proximal end functionally associated with the sprayer and a distal end, the feeder tube configured to define a conduit for a sprayable composition from the distal end of the feeder tube to the sprayer; and c) an anchor functionally associated with the distal end of the feeder tube, wherein the anchor and the feeder tube are configured so that when properly deployed in the body of a subject, the feeder tube passes through the pyloric sphincter of the subject to maintain the sprayer in the duodenum of a subject.

According to some embodiments, the device further comprises an increased-diameter feature near the distal of the feeder tube, configured to assist in preventing the sprayer from passing through the pyloric sphincter into a stomach of a subject when properly deployed. According to some embodiments, the increased-diameter feature comprises the sprayer. According to some embodiments, the increased-diameter feature is expandable, easing deployment in a small-diameter conformation and preventing the passage back through the pyloric sphincter in an increased diameter conformation.

According to some embodiments, the feeder tube and the anchoring component are configured so that when properly deployed in the body of a subject, the feeder tube passes through the pyloric sphincter of the subject to maintain the sprayer in the superior portion of the duodenum of a subject.

According to some embodiments, the device further comprises a pressure generator functionally associated with the feeder tube which is configured to force sprayable composition out through the sprayer as a spray upon actuation of the pressure generator. Depending on the embodiments, the pressure generator is configured for deployment inside the gastrointestinal tract, for deployment in the abdominal cavity, for subcutaneous deployment, for deployment outside the body of the subject, or for deployment elsewhere.

According to some embodiments, the device further comprises a sprayable composition reservoir functionally associated with the pressure generator, wherein the sprayable composition forced out through the sprayer by the pressure generator is sprayable composition held in the reservoir. Depending on the embodiment, the pressure generator is configured for deployment inside the gastrointestinal tract, for deployment in the abdominal cavity, for subcutaneous deployment, for deployment outside the body of the subject, or for deployment elsewhere.

According to some embodiments, where the pressure generator and/or the sprayable composition reservoir are not configured to be deployed in the gastrointestinal tract (e.g., when configured for deployment in the abdominal cavity, subcutaneously or outside of the body of a subject), the feeder tube is configured to pass through an opening in a wall of the gastrointestinal tract.

In some embodiments, the sprayer comprises at least one orifice. In some embodiments, at least one orifice is a nozzle. In some embodiments, at least one orifice is a slit. In some embodiments, the at least one the orifice is configured to direct a spray towards a luminal wall of a gastrointestinal tract in which the sprayer is deployed (e.g., the sprayer has an axis and the orifice is configured to direct a spray away from an axis of the sprayer). In some embodiments, the sprayer comprises at least two orifices configured to direct a spray towards a luminal wall of a gastrointestinal tract in which the sprayer is deployed (e.g., the sprayer has an axis and the orifices are configured to direct a spray away from an axis of the sprayer) each in a different direction away from an axis of the sprayer.

In some embodiments, at least one (preferably all) of the orifice is configured to function as a valve allowing the sprayable composition to be forced out through the sprayer upon actuation of the pressure generator but substantially preventing passage of fluids through the orifice when the pressure generator is not actuated.

In some embodiments, the device is configured so that, when deployed, the sprayer is suspended in the lumen of the gastrointestinal tract.

In some embodiments, the device is configured so that, when deployed, a portion of the sprayer is near to or contacting a part of the luminal wall of the gastrointestinal tract. In some embodiments, the sprayer is configured (for example by positioning and shape of orifices) to spray towards a luminal wall in proximity of the part of the luminal wall that the portion of the sprayer contacts or is near to.

In some embodiments, a sprayer of the device is configured to spray a region of no less than 1 cm long of a luminal wall in which the sprayer is deployed, for example by including a plurality of orifices along a length of the sprayer or by providing orifices having a relatively large arc with respect to the length of the gastrointestinal tract. In some embodiments the length of the region sprayed is of no less than 2 cm, of no less than 3 cm and even of no less than 4 cm.

In some embodiments, the device is configured so that the sprayer is deployable at a specific location in a gastrointestinal tract, for example in the esophagus, stomach, antrum, antral sphincter, fundus, pylorus, small intestine, duodenum, jejunum, ileum, large intestine, caecum, vermiform appendix, colon, ascending colon, transverse colon, descending colon, sigmoid flexure or rectum. In some embodiments, the device is provided with a marker (e.g., a feature or component) that is observable and allows determination of the location of the sprayer in the gastrointestinal tract. In some embodiments, a marker is radio-opaque. In some embodiments, a marker is ultrasound opaque.

In some embodiments, the device further comprises e) an anchor, configured to anchor the sprayer at a specific location in the gastrointestinal tract.

In some embodiments, the sprayer is in fluid communication with the pressure generator through a feeder tube, allowing transport of a fluid from the pressure generator to the sprayer. Generally the size of the bore of the feeder tube is determined by the length of the feeder tube from the pressure generator, the properties of the sprayer, the properties of the pressure generator and the viscosity of the composition which the device is configured to spray. In some embodiments, the outer diameter of the feeder tube is no more than about 4 mm, no more than about 3 mm and even no more than 2 mm. In some embodiments, a feeder tube is configured, at least in part, to assist in anchoring and/or positioning the sprayer in a desired location in the gastrointestinal tract. For example, in some embodiments, at least portion of a feeder tube is coiled so as, when deployed in the gastrointestinal tract, the coiled portion of the feeder tube presses against the luminal wall of the gastrointestinal tract to position or anchor the sprayer.

In some embodiments, the pressure generator is selected from the group consisting of spring powered pressure generators, motor powered pressure generators and gas pressure powered pressure generators. In some embodiments, the device further comprises a power supply unit for providing power for operation of the pressure generator. Typical power supply units include power storage units (especially rechargeable power storage units such as rechargeable batteries) and power generation units.

In some embodiments, the device further comprises d) a composition reservoir functionally associated with the pressure generator and the pressure generator is configured to force a sprayable composition held in the reservoir from the reservoir out through the sprayer as a spray upon the actuation. In some embodiments, the reservoir is configured for deployment inside the body of a subject and in some embodiments is even configured to be recharged when deployed inside the body. In some embodiments, the reservoir is configured for deployment in a gastrointestinal tract, preferably in a stomach. In some embodiments, the reservoir is configured for recharging with composition when deployed in a gastrointestinal tract. In some embodiments, the reservoir is configured for implantation in the body of a subject, preferably subcutaneously. In some embodiments, the reservoir is configured for recharging with composition when implanted in the body. In some embodiments, the reservoir is configured for deployment outside of the body of a subject.

In some embodiments, a device of the present invention further comprises a pharmaceutical composition including a peptide active agent (especially having no more than 40 amino residues, no more than 30 amino acid residues and even no more than 20 amino acid residues) and a pharmaceutically acceptable carrier held in a reservoir. In some embodiments the active agent is a peptide hormone, an analogue thereof or a derivative thereof. In some embodiments, the active agent is configured to interact with chemoreceptors apparent on luminal walls of a portion of a gastrointestinal tract in which the sprayer is to be deployed.

In some embodiments, the actuator is configured to actuate the pressure generator so as to dispense a specified dose of sprayable composition. In some embodiments the dose is a fixed dose. In some embodiments, the device further comprises a dosage adjusting mechanism functionally associated with the actuator and/or with the pressure generator.

In some embodiments, the device further comprises f) an event detector functionally associated with the actuator, configured so that as a result of detection of an event of significance for administration of an active agent, the event detector triggers the actuator.

In some embodiments, the event is a physiological change. Typical physiological changes for example gastrointestinal tract activity indicative of an event such as food ingestion, hunger, anginal attack, arrhythmia, an asthma attack or an allergic reaction especially a systemic allergic reaction.

In some embodiments, the event detector comprises an electrode configured for deployment in the body.

In some embodiments, the device comprises a timer functionally associated with the actuator and with the event detector and the timer is configured to trigger the actuator a specified period of time subsequent to the detection of the event by the event detector.

In some embodiments, the device comprises a timer functionally associated with the actuator and, for example, the timer is configured to periodically trigger the actuator.

In some embodiments, a timer is configured to trigger the actuator for a specified period of time. In some embodiments, a timer is adjustable, that is, the period of time over which a sprayable composition is administered or the time delay after detection of an event which the sprayable composition is administered is changeable.

In some embodiments, the device for administering a sprayable pharmaceutical composition including an active agent to a luminal wall of the gastrointestinal tract of a subject, comprises: a) a sprayer configured for substantially fixed deployment in a gastrointestinal tract, the sprayer comprising at least one orifice configured to direct a spray towards a portion of the luminal wall of a gastrointestinal tract (preferably of the duodenum) in which the sprayer is deployed; b) a pressure generator configured to dispense a dose of sprayable composition out through the sprayer as a spray upon actuation; c) an actuator for actuating the pressure generator upon being triggered; d) a composition reservoir functionally associated with the pressure generator so that the pressure generator is configured to force sprayable composition from the reservoir out through the sprayer as a spray upon actuation; and further comprising a sprayable pharmaceutical composition including a peptide active agent (especially having no more than 40 amino residues, no more than 30 amino acid residues and even no more than 20 amino acid residues) and a pharmaceutically acceptable carrier held in the reservoir. In some embodiments, the device further comprises d) an event detector functionally associated with the actuator, configured so that as a result of detection of an event of significance for administration of an active agent, the event detector triggers the actuator. In some embodiments, the event is a physiological change indicative of food ingestion and/or hunger. In some embodiments, the active agent is configured to interact with chemoreceptors apparent on luminal walls of the portion of the gastrointestinal tract. In some embodiments, the active agent is a peptide hormone, an analogue thereof or a derivative thereof. In some embodiments, the active agent is a gastrointestinal satiety agent, such as a CCK analogue or CCK derivative, such as CCK-8.

It has been found that gastrointestinal administration of composition including peptide active ingredients such as peptide hormones for treating medical conditions without the peptides being substantially digested in the gastrointestinal tract before having a desired physiological effect may be useful.

Thus, there is also presented a method of treatment, comprising: a) providing a sprayable pharmaceutical composition which comprises a peptide active agent and a pharmaceutically acceptable carrier; and b) administering the composition to a subject in need thereof by spraying the composition in a part of the gastrointestinal tract of the subject so that the active agent interacts with the luminal wall of the gastrointestinal tract thereby causing a beneficial effect. In some examples the luminal wall is the luminal wall of the duodenum of the subject. In some examples, the peptide is a peptide hormone, an analogue thereof or a derivative thereof. In some examples, at least some of the beneficial effect results from the interaction of the active agent with chemoreceptors apparent on the luminal wall. In some examples, the peptide has no more than 40 amino residues, no more than 30 amino acid residues and even no more than 20 amino acid residues. In some examples, the active agent is a peptide hormone, an analogue thereof or a derivative thereof. In some embodiments, the active agent is a gastrointestinal satiety agent. In some examples, the active agent is a CCK analogue or CCK derivative such as CCK-8.

Thus, there is also presented a method of treatment, comprising: a) providing a sprayable pharmaceutical composition which comprises a satiety factor or an analogue thereof or a derivative thereof as an active agent and a pharmaceutically acceptable carrier; and b) administering the composition to a subject in need thereof by spraying the composition in a portion of the gastrointestinal tract of the subject so that the active agent interacts with chemoreceptors apparent on the luminal wall of the portion of the gastrointestinal tract, thereby causing a beneficial effect. In some examples the luminal wall is the luminal wall of the duodenum of the subject. In some examples the subject suffers from a disorder selected from the group consisting of obesity, bulimia, eating disorders, overeating, diabetes-related obesity and metabolic syndrome. In some cases the beneficial effect is a reduction of calories consumed and/or a reduction of the amount food consumed. In some examples, the satiety factor is CCK or an analogue thereof or a derivative thereof such as CCK-8.

Thus according to the present invention as defined in claim 11, there is also provided a sprayable composition comprising a peptide (especially a peptide having no more than 40 amino residues, no more than 30 amino acid residues and even no more than 20 amino acid residues) as an active agent for use in the treatment of a subject by gastrointestinal administration (especially duodenal administration). In some embodiments, the peptide is a peptide hormone, an analogue thereof or a derivative thereof.

Thus, according to some embodiments of the present invention there is also provided for the use of a peptide (especially having no more than 40 amino residues, no more than 30 amino acid residues and even no more than 20 amino acid residues) as an active agent for a sprayable pharmaceutical composition (that is to say a medicament) for use in the treatment of a subject by gastrointestinal administration (especially duodenal administration), wherein the peptide is configured to interact with chemoreceptors apparent on the surface of the gastrointestinal tract. In some embodiments, the treatment comprises reduction of calorie intake by the subject and or reduction of the amount of food consumed by a subject. In some embodiments, the subject suffers from a disorder selected from the group consisting of obesity, bulimia, eating disorders, overeating, diabetes-related obesity and metabolic syndrome. In some embodiments, the peptide is a peptide hormone, an analogue thereof or a derivative thereof. In some embodiments, the peptide is a gastrointestinal satiety agent, such as a CCK analogue or CCK derivative such as CCK-8.

In some embodiments, the sprayable composition is configured for treating a condition selected from the group consisting of obesity, bulimia, eating disorders, overeating, diabetes-related obesity, metabolic syndrome, inflammatory bowel disease, infections such as of helicobacter pylori, cardiovascular pathologies such as angina or arrhythmia, asthma and allergies.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

As used herein, the terms "comprising" and "including" or grammatical variants thereof are to be taken as specifying the stated features, integers, steps or components but do not preclude the addition of one or more additional features, integers, steps, components or groups thereof. This term encompasses the terms "consisting of" and "consisting essentially of'.

The phrase "consisting essentially of" or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed composition, device or method.

Herein, the term "active agent" is meant to include chemical, biological or pharmaceutical materials including any natural or synthetic chemical or biological substance that influences a cell, an organ or organism to which the material is administered. Typical active agents include but are not limited to active pharmaceutical ingredients, antibodies, antigens, biological materials, chemical materials, chemotherapeutic agents, diagnostic agents, DNA, drugs, dyes, enzymes, foodstuffs, hormones, immunogenes, ligands, liposomes, markers, nanoparticles, nucleic acids, nutrients, physiological media, proteins, radio-labeled markers, RNA, selective toxins, therapeutic monoclonal antibodies, toxins and vaccines and especially peptides and peptide hormones.

BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of some embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

FIGS. 1A, 1B, 1C and 1D are schematic depiction of a first embodiment of a device of the present invention;
FIG. 2 is a schematic depiction of the device of Figures 1 deployed in a gastrointestinal tract;
FIG. 3 is a schematic depiction of an embodiment of a second embodiment of a device of the present invention deployed in a gastrointestinal tract;
FIGS. 4A, 4B and 4C are schematic depiction of a second embodiment of a device of the present invention;
FIGS. 5A and 5B are schematic depictions of head-on cross sections of embodiments of sprayers suitable for implementing the teachings of the present invention;
FIGS. 6A, 6B, 6C and 6D are schematic depictions of side cross sections of embodiments of sprayers suitable for implementing the teachings of the present invention;
FIG. 7 is a schematic depiction of a device used to test the teachings of the present invention;
FIG. 8 is a graph displaying the effect of administration of CCK-8 in accordance with the teachings of the present invention on gallbladder contraction;
FIG. 9 is a graph displaying the effect of administration of CCK-8 in accordance with the teachings of the present invention on caloric consumption;
FIG. 10 is a graph displaying the effect of administration of CCK-8 in accordance with the teachings of the present invention on food consumption;
FIG. 11 is a graph displaying the effect of administration of CCK-8 in accordance with the teachings of the present invention on food taken; and
FIGS. 12A-12E depict an embodiment of a device of the present invention where a feeder tube passes from outside the body of a subject, into the stomach cavity, through the pyloric sphincter to a sprayer deployed in the duodenum.

DESCRIPTION OF EMBODIMENTS

Some embodiments of the present invention are of devices for the administration of an active agent for treating a condition. Specifically, some embodiments of the the devices of the present invention relate to administration of a sprayable pharmaceutical composition including an active agent by deploying a sprayer at a specific location in the gastrointestinal tract and, when needed, spraying the composition through the sprayer at a luminal wall of the gastrointestinal tract. Some embodiments of the present invention also relate to the use of peptides, such as peptide hormones, such as CCK, CCK analogues and derivatives thereof in the preparation of pharmaceutical compositions (medicaments).

Typically a sprayer of the present invention is deployed in a specific location of a gastrointestinal tract of a subject suffering from a condition, preferably so that the sprayer is substantially fixed in place and a sprayable pharmaceutical composition comprising an active agent effective in treating the condition is provided. The specific location is selected as being a location that is in preferred, ideal or in some way advantageous for administration of the active agent, for example the location is where the administered active agent is ideally absorbed, where chemoreceptors sensitive to the active agent are found or where administration exhibits the fewest side effects.

Although not wishing to be held to any one theory, it is believed that administering a composition by spraying the composition onto a surface where receptors are found (for example, receptors found on the duodenal lumen) increases the availability of an active agent to the receptors, increases absorption and more evenly disperses the composition.

When necessary, a dose of a sprayable pharmaceutical composition (in some embodiments a fluid composition, in some embodiments a liquid composition, especially a non-viscous liquid composition) is sprayed through the sprayer against a portion of a luminal wall of the gastrointestinal tract of the subject thereby administering the active agent so as to treat the condition.

By treating the condition are included, but not limited to, curing the condition, treating the condition, preventing the condition, treating symptoms of the condition, curing symptoms of the condition, ameliorating symptoms of the condition, treating effects of the condition, ameliorating effects of the condition, and preventing results of the condition.

By dose is meant a pharmaceutically effective amount of sprayable pharmaceutical composition that includes the active agent. By "pharmaceutically effective amount" is meant describes an amount of the composition that is sufficient to lead to a desired effect in the condition being treated, but low enough to avoid significant side effects, within the scope of sound judgment of a health care professional such as a medical doctor. The effective amount of the composition may vary with the particular area being treated, the age and physical condition of the subject being treated, the severity of the condition, the duration of the treatment, the nature of concurrent therapy, the specific active agent employed, the particular carrier utilized, and like factors within the knowledge and expertise of one skilled in the art.

As used herein a "pharmaceutical composition" refers to a preparation of one or more of the active agents with other components such as physiologically suitable carriers and excipients. The purpose of a pharmaceutical composition is to facilitate administration of a compound to a subject. Formulation of a sprayable pharmaceutical composition and determination of a suitable dose is within the ability of one of average skill in the art using techniques with which one of average skill is familiar which are discussed in numerous reference works such as Remington's Pharmaceutical Science 15th Edition and generally includes mixing an amount of the active agent with another material or materials, such as excipients and carriers.

Hereinafter, the phrases "physiologically acceptable carrier" and "pharmaceutically acceptable carrier" which may be interchangeably used refer to a carrier or a diluent that does not cause significant irritation to a subject and does not abrogate the biological activity and properties of the administered active agent. An adjuvant is included under these phrases.

Herein the term "excipient" refers to an inert substance added to a pharmaceutical composition to further facilitate administration of an active agent. Examples, without limitation, of excipients include calcium carbonate, calcium phosphate, various sugars and types of starch, cellulose derivatives, gelatin, vegetable oils and polyethylene glycols.

Pharmaceutical compositions used in implementing the teachings of the present invention may be formulated in conventional manner using one or more physiologically acceptable carriers comprising excipients and auxiliaries, which facilitate processing of the active entities into pharmaceutical compositions. Suitable techniques are described in "Remington's Pharmaceutical Sciences," Mack Publishing Co., Easton, PA, latest edition, which is incorporated herein by reference. For example, pharmaceutical compositions of the present invention may be manufactured by one or more processes that are well known in the art, e.g., mixing, blending, homogenizing, dissolving, granulating, emulsifying, encapsulating, entrapping and lyophilizing processes. For example, sprayable compositions of the present invention may include aqueous solutions, in some embodiments in physiologically compatible buffers such as Hank's solution, Ringer's solution, or physiological salt buffer. For transmucosal administration, penetrants appropriate to the barrier to be permeated are used in the formulation. Such penetrants are generally known in the art.

Pharmaceutical compositions suitable for use in the context of the present invention generally include compositions comprising active agents in an amount effective to achieve the intended purpose, for example in some embodiments a therapeutically effective amount means an amount of active agent (e.g., a satiety drug or an anti-food absorption drug) effective in reducing appetite. Determination of a therapeutically effective amount is well within the capability of those skilled in the art, especially in light of the detailed disclosure provided herein. When implementing the teachings of the present invention, a therapeutically effective amount or dose can be estimated initially from animal models such as monkey or pigs. For example, a dose can be formulated in animal models to achieve a desired concentration or titer. Such information can be used to more accurately determine useful doses in humans.

For example, a dose of CCK-8 can be in the range of 0.04 to 0.4 micrograms per kg body weight. Thus, for an individual who weighs 125 kg, such a dose can be for example of 10 micrograms CCK-8.

Exemplary conditions from which the subject suffers for which some embodiments of the method of the present invention may be useful include, but are not limited to obesity, bulimia, eating disorders, overeating, diabetes-related obesity, metabolic syndrome, inflammatory bowel disease, helicobacter pylori infection, cardiovascular pathologies, asthma and allergy.

In some examples, the method is a topical method of administration, that is to say the active agent acts for example by interacting with chemoreceptors apparent on or in the gastrointestinal tract luminal wall or the active agent acts by treating a pathology apparent on the gastrointestinal tract luminal wall In some cases, the method is a systemic method of administration, that is to say the active agent is absorbed by the organism through the gastrointestinal tract in order to act.

In some examples, the necessity of administration of the active agent is part of a regular administration protocol, for example a prophylactic or maintenance dose, for example for the treatment of a chronic condition. In some cases, a dose of the active agent is administered periodically, that is, doses are administered according to a substantially fixed schedule. Depending on the severity and responsiveness of the condition to be treated, a given treatment regime may by chronic, last from several days, several weeks or until a desired effect is achieved. It will be appreciated that an efficient dose can be adjusted to the treated individual based. That said, the treatment regime (e.g., dosage, frequency) of a composition be administered will, of course, be dependent on the subject being treated, the severity of the subject and the judgment of the responsible health-care professional.

In some embodiments, administration of the sprayable composition is initiated and performed manually (e.g., by a caregiver, a health-care professional or the subject self) while in some embodiments administration is initiated and performed automatically.

In some embodiments, administration of the active agent is event-driven, that is a dose of active agent is administered subsequent to the detection of some event of significance for administration of the active agent, for example physiological changes. In some embodiments related to eating disorders typical significant events include detection of muscle activity (e.g., of the stomach), pressure (e.g., caused by stomach contractions or resulting from food entering the stomach or esophagus), change of chemical composition such as pH (e.g., from the release of enzymes, acids or other gastric juices), body temperature and electrical currents in the vagus nerves or pancreas when a person is hungry or consuming food. In some embodiments related to the treatment of a subject suffering from a cardiovascular condition typical significant events include detection of an anginal attack or arrhythmia. In some embodiments related to the treatment of a subject suffering from asthama typical significant events include detection of an asthma attack. In some embodiments related to the treatment of a subject suffering from an allergy typical significant events include detection of an allergic reaction, especially a systemic allergic reaction.

In some embodiments, detection of an event is manual, that is to say, includes at least one step performed by a person, such as a caregiver, a health-care professional or the subject self. For example, a subject suffering from asthma identifies an asthmatic attack and initiates active agent administration. In some embodiments, detection of an event is automatically performed, for example with the help of an event detector that is functionally associated with the sprayer.

In some cases, the method further comprises functionally associating a reservoir holding the sprayable composition with the sprayer, allowing the method to be performed unobtrusively, autonomously, automatically and with minimal inconvenience for the subject. In some examples, functionally associating the reservoir with the sprayer comprises deploying the reservoir in the gastrointestinal tract, for example, in the stomach. In some embodiments, functionally associating the reservoir with the sprayer comprises implanting the reservoir in the body of the subject, for example, subcutaneously.

The passage of an active agent orally administered in accordance with the prior art through the gastrointestinal tract is a substantially continuous process where the administered active agent is diluted in the gastrointestinal fluids. Thus, the concentration of the active agent in the gastrointestinal fluid is indeterminate and varies as a result of many factors including the volume of the fluid as well as the composition of the fluid which may include fibers and other molecules which may absorb, adsorb or otherwise influence the active agent. Further, the pH of the gastrointestinal fluid changes the ratio of ionized/un-ionized active agent. In addition, the varying rate of peristalsis that changes the flow rate through the gastrointestinal tract combined with the variable dilution makes the effective concentration of an active agent at the lumen wall indeterminate and difficult to control. The result is that an administered dose of an active agent may often be too low (leading to insufficient efficacy) or too high (leading to any one of numerous negative effects) while a significant proportion of an active agent is carried through the gastrointestinal tract past sites such as chemoreceptors where the active agent is effective.

The teachings of some embodiments of the present invention are based, at least in part, on the discovery that spray-administration of a sprayable composition containing an active agent at a specific location of the gastrointestinal tract, especially at carefully selected moments, is an unexpectedly effective manner of administering the active agent, the spraying overcoming many of the challenges of gastrointestinal tract delivery of active agents.

Although not wishing to be held to any one theory, it is believed that administration in accordance with the teachings of the present invention brings an administered active agent in contact with a luminal wall of the gastrointestinal tract where the pharmaceutical or other beneficial activity of the active agent occurs at a significantly more controlled effective dosage in a relatively well defined chemical and physical environment as the spraying reduces mixing with the gastrointestinal liquids. Thus, the teachings of the present invention apparently provide for administering an active agent at a controlled concentration under controlled chemical conditions to a well-determined location in the gastrointestinal tract. As a result, some embodiments of the present invention allow administration of a lesser amount of active agent reducing potential side effect. Further, the chance of overdosing is reduced as in some embodiments, the time between administration and an actual effect is generally short.

An additional unexpected effect is the surprising efficacy with which peptides, such as peptide hormones, are administered. It is known that it is challenging to administer peptides as active agents through the gastrointestinal tract as these are quickly digested and rendered ineffective by the chemical conditions inside the gastrointestinal tract. Further, peptide absorption is generally affected by pH due to the ease with which peptides are ionized. The present invention therefore provides an unexpectedly convenient way for administering peptide active agents in pharmaceutical compositions and in medicaments. Further, in some embodiments the present invention allows for the use of active agents such as peptides in the preparation of pharmaceutical compositions (medicaments), especially pharmaceutical compositions (medicaments) that are substantially devoid of peptidase inhibitors and the administration of such pharmaceutical compositions in accordance with the teachings of the present invention.

The term "peptide" as used herein encompasses native peptides and their analogues (either degradation products, synthetically synthesized peptides or recombinant peptides) and peptidomimetics (typically, synthetically synthesized peptides), as well as peptoids and semipeptoids which are peptide analogs, which may have, for example, modifications rendering the peptides more stable while in a body or more capable of penetrating into cells, especially peptides that are not longer than 40, not longer than 30 and even not longer than 20 amino acid residues long. Such modifications include, but are not limited to N terminus modification, C terminus modification, peptide bond modification, including, but not limited to, $CH_2$-NH, $CH_2$-S, $CH_2$-S=O, O=C-NH, $CH_2$-O, $CH_2$-$CH_2$, S=C-NH, CH=CH or CF=CH, backbone modifications, and residue modification. Methods for preparing peptidomimetic compounds are well known in the art and are specified, for example, in Quantitative Drug Design, C.A. Ramsden Gd., Chapter 17.2, F. Choplin Pergamon Press (1992), which is incorporated by reference as if fully set forth herein.

Peptide bonds (-CO-NH-) within the peptide may be substituted, for example, by N-methylated bonds (-N($CH_3$)-CO-), ester bonds (-C(R)H-C-O-O-C(R)-N-), ketomethylen bonds (-CO-$CH_2$-), *-aza bonds (-NH-N(R)-CO-), wherein R is any alkyl, e.g., methyl, carba bonds (-$CH_2$-NH-), hydroxyethylene bonds (-CH(OH)-$CH_2$-), thioamide bonds (-CS-NH-), olefinic double bonds (-CH=CH-), retro amide bonds (-NH-CO-), peptide derivatives (-N(R)-$CH_2$-CO-), wherein R is the "normal" side chain, naturally presented on the carbon atom. These modifications can occur at any of the bonds along the peptide chain and even at several (2-3) at the same time.

Natural aromatic amino acids, Trp, Tyr and Phe, may be substituted for synthetic non-natural acid such as TIC, naphthylelanine (Nol), ring-methylated derivatives of Phe, halogenated derivatives of Phe or o-methyl-Tyr.

In addition to the above, the peptides used in present invention may also include one or more modified amino acids or one or more non-amino acid monomers (e.g., fatty acids and complex carbohydrates.

The term "amino acid" or "amino acids" is understood to include the 20 naturally occurring amino acids; those amino acids often modified post-translationally in vivo, including, for example, hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids.

Peptides used in implementing the teachings of the present invention may be linear or cyclic.

Peptides used in implementing the teachings of the present invention may be synthesized by any techniques that are known to those skilled in the art of peptide synthesis. For solid phase peptide synthesis, a summary of the many techniques may be found in J. M. Stewart and J. D. Young, Solid Phase Peptide Synthesis, W. H. Freeman Co. (San Francisco), 1963 and J. Meienhofer, Hormonal Proteins and Peptides, vol. 2, p. 46, Academic Press (New York), 1973. For classical solution synthesis see G. Schroder and K. Lupke, The Peptides, vol. 1, Academic Press (New York), 1965. In general, such methods include the sequential addition of one or more amino acids or suitably protected amino acids to a growing peptide chain. Normally, either the amino or carboxyl group of the first amino acid is protected by a suitable protecting group. The protected or derivatized amino acid can then either be attached to an inert solid support or utilized in solution by adding the next amino acid in the sequence having the complimentary (amino or carboxyl) group suitably protected, under conditions suitable for forming the amide linkage. The protecting group is then removed from this newly added amino acid residue and the next amino acid (suitably protected) is then added, and so forth. After all the desired amino acids have been linked in the proper sequence, any remaining protecting groups (and any solid support) are removed sequentially or concurrently, to afford the final peptide compound. By simple modification of this general procedure, it is possible to add more than one amino acid at a time to a growing chain, for example, by coupling (under conditions which do not racemize chiral centers) a protected tripeptide with a properly protected dipeptide to form, after deprotection, a pentapeptide and so forth. Further description of peptide synthesis is disclosed in U.S. Patent No. 6,472,505.

A preferred method of preparing the peptide compounds of the present invention involves solid phase peptide synthesis.

Large scale peptide synthesis is described by Andersson in Biopolymers 2000;55(3):227-50.

In some embodiments, the present invention helps in the effective administration of gastrointestinal satiety agents or derivatives thereof or analogs thereof that mimic the physiological action of the gastrointestinal satiety agent. Such active agents include naturally occurring or synthetic hormones, peptides, neurotransmitters or mimetic thereof, that are capable of directly stimulating the region responsive to the satiety agent (e.g., the duodenum). Specific active agents for which administration using the teachings of the present invention is exceptionally useful include but are not limited to peptide hormones, CCK (GenBank Accession No. NP_000720), Bombesin, Gastrin releasing peptide (GRP), glucagon, Enterostatin, Ghrelin, GLP-1 (glucagon-like peptide) (Bojanowska E., 2005, Med. Sci. Monit. 11:RA271-8; BYETTA™ (exenatide)), PYY (le Roux CW., et al., 2005, Endocrinology. 2005 Sep 15; GenBank Accession No. NP_004151), Oxyntomodulin (OXY, OXM; GenBank Accession No. P01275; Stanley S., et al., 2004, Am. J. Physiol. Gastrointest. Liver Physiol. 286(5): G693), Apo IV (naturally occurring apoprotein Qin X, Tso P 2005, Curr Drug Targets. 6 (2):145-51), GII81771X (GSK), anti Ghrelin agents (Kobelt P., Gut. 2005 Jun 30; SPIEGELMER NOX-B11), PP (Miskowiak J, et al., 1985, Regul. Pept. 12: 231-6) and derivatives and analogs thereof such as CCK-4 (Trp-Met-Asp-Phe), CCK-8 (Asp-Tyr($SO_3$H)-Met-Gly-Trp-Met-Asp-Phe) analogues of CCK), CCK analogs ((Sincalide by Bracco Diagnostics or Squibb Diagnostics), GSK - GW7176, GW 5283, GW7854 and Pfizer PW170292)), CCK receptor agonists (e.g., 1,5-benzodiazepines, PD 170292, SR 146131) and/or activator molecules of the CCK-A receptor (JMV 180; Archer-Lahlou E, et al., 2005, J. Biol. Chem., Vol. 280: 10664-10674), and PYY analogs (e.g., PYY(1-36), PYY(3-36), PYY(9-36), PYY (14-36), PYY(22-36), and PYY(27-36)).

Some embodiments of the present invention actualize a central concept taught in the PCT patent application published as WO 2006/035446 of the Applicant by the unexpected discovery that stimulation of gastrointestinal chemoreceptors with an active agent such as an API (e.g., a peptide hormone) is exceptionally effective when the active agent is administered by spraying a composition including the active agent at the luminal wall of the gastrointestinal tract where the chemoreceptors are found. The spray provides for a quick administration of a layer of composition including an active agent over a large surface area of the luminal wall with relatively little composition wasted by dilution in the gastrointestinal fluid.

Some embodiments of the present invention have further significant advantages. As a dose is not necessarily prepackaged, but is rather determined by an amount of sprayable composition sprayed, in some embodiments a dose administered to a subject is personalized and can be adjusted for increased efficacy. As compositions are administered according to the teachings of the present invention directly in the gastrointestinal tract and do not pass the mouth and other sensitive tissue, compositions different than those known in the art, for example compositions that are bitter, oily or otherwise unpalatable may be administered. In some embodiments, compositions including low solubility active agents are administered by administering greater volumes of a composition or by administering compositions having a higher than usual organic solvent (e.g., ethanol, propylene glycol) content. In some embodiments, complex administration protocols may be formulated (e.g., multiple times daily or at exact intervals or including two or more separate active agents). Some embodiments of the present invention including automatic administration and especially also including automatic administration triggered by automatic event detection virtually guarantee absolute patient compliance. Some embodiments of the present invention are' exceptionally suitable for chronic administration, by ensuring long-term patient compliance and by efficient use of a given amount of active agent to reduce side effects. In some embodiments of the present invention including automatic administration of a composition triggered by automatic event detection, treatment is exceptionally effective as an event may be detected before significant damage is done and a precise dose of active agent is administered without overdosing.

An aspect of the present invention is of a device suitable for administering a sprayable pharmaceutical composition including an active agent to a luminal wall of the gastrointestinal tract, for example, for implementing some embodiments of the method of the present invention. Generally, some embodiments of a device of the present invention comprise: a) a sprayer configured for deployment in a gastrointestinal tract; b) a pressure generator (e.g., a pump, such as a mechanical pump, an electrical pump, an pressurized gas-powered pump) configured to dispense a dose of sprayable composition out through the sprayer as a spray upon actuation; and c) an actuator for actuating the pressure generator upon being triggered.

Some embodiments of the sprayer of a device of the present invention include one or more orifices that are configured to direct a spray in one or more directions towards a luminal wall of a gastrointestinal tract in which the sprayer is deployed.

Some embodiments of a device of the present invention comprise an event detector to automatically detect an event of significance for administration of an active agent and, upon detection of such an event, to automatically trigger the actuator so as to administer a dose of the sprayable pharmaceutical composition.

Some embodiments of a device of the present invention comprise a composition reservoir functionally associated with the pressure generator and the pressure generator is configured to force sprayable composition from the reservoir out through the sprayer as a spray upon the actuation. In some embodiments, the reservoir is configured for deployment in a gastrointestinal tract, preferably in a stomach. This allows the device to be deployed in the gastrointestinal tract of a subject unobtrusively and with minimal inconvenience and to function automatically. In some embodiments, the reservoir is configured for implantation in the body of the subject, preferably subcutaneously. This allows the device to be deployed in the body of a subject unobtrusively and with minimal inconvenience and to function automatically.

The principles of the device of the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings. The invention is capable of other some embodiments or of being practiced or carried out in various ways. Also, it is to be understood that the phraseology and terminology employed herein is for the purpose of description and should not be regarded as limiting.

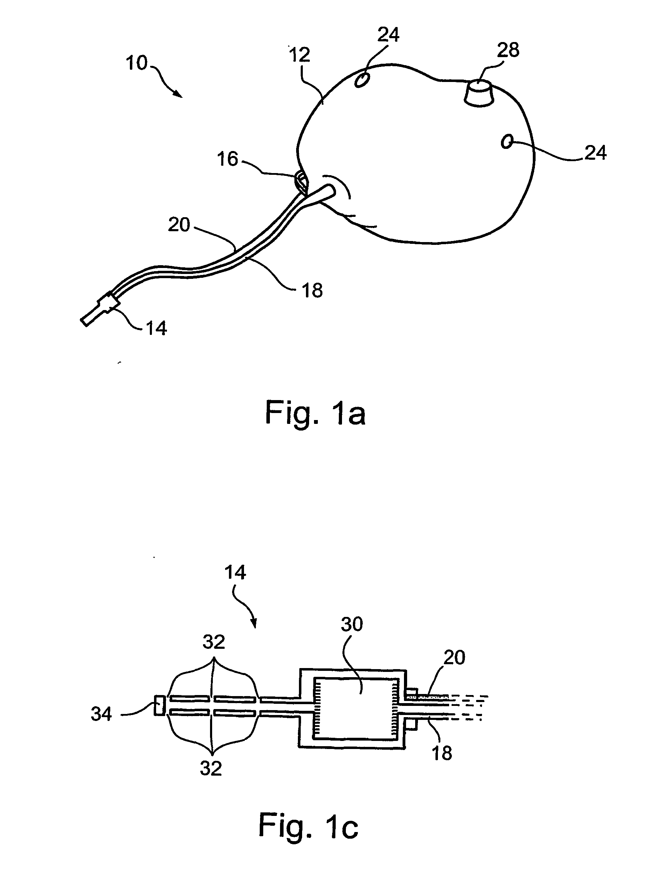

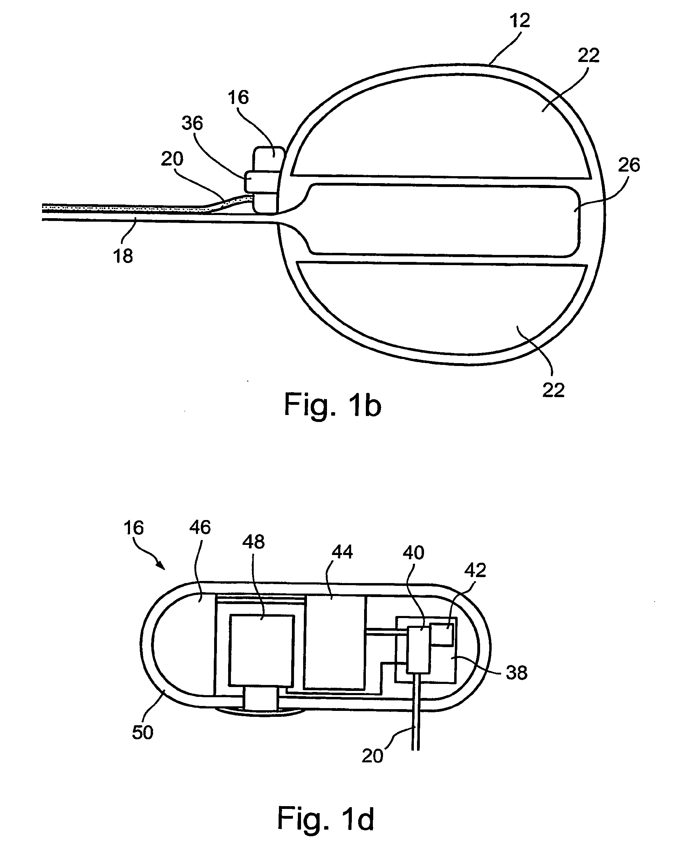

In Figures 1A, 1B, 1C and 1D are depicted an embodiment of the device of the present invention, device **10** configured for treating conditions relating to eating disorders for which administration of satiety agents may be beneficial. Device **10** comprises a number of components including reservoir **12**, spray head **14**, control unit **16**, feeder tube **18** and power line **20**.

Reservoir **12,** depicted in cross section in Figure 1B, is substantially a three-chambered elastic balloon configured for deployment in the stomach and is similar in construction and made of materials known in the art of intragastric balloons, e.g., BioEnterics® Intragastric Balloon System (Inamed Health, a division of Allergan, Santa Barbara, CA, USA) or as discussed in the PCT patent application published as WO 2006/035446. Reservoir **12** comprises two space-filling chambers **22** each associated with a charging port **24** and a composition chamber **26** associated with a charging port **28** and in fluid communication with feeder tube **18**. Chambers **22** and **26** are ordinarily in a collapsed state but stretch outwards when a fluid, such as a gas or liquid, is introduced into a chamber through a respective charging port **24** or **28**. When entirely filled, space-filling chambers **22** occupy a volume of approximately 300 ml. When entirely filled, composition chamber **26** occupies a volume of approximately 100 ml.

Spray head **14,** depicted in cross section in Figure 1C, comprises a pressure generator **30** (an electrically powered pump, for example similar to pumps used in the field of insulin administration) that takes a sprayable composition from feeder tube **18** and forces the composition out as a spray through nozzles **32** (six nozzles **32** are depicted in Figure 1C) of sprayer **34**. As is seen in Figure 1C, nozzles **32** are positioned and configured so that a spray exiting from nozzles **32** is directed away from the axis of sprayer **34** and thus sprayer **34** is configured to direct a spray towards the luminal wall of a gastrointestinal tract in which sprayer **34** is deployed, where nozzles **32** are arranged to spray in at least two different directions away from the axis.

Control unit **16** is depicted in Figures 1A and, 1B secured to reservoir **12** with an elastic band **36**. In Figure 1D components of control unit **16** are depicted: actuator **38** comprising controller **40** and timer **42,** power storage unit **44,** power storage unit charger **46,** event detector **48** and casing **50**.

Event detector **48** comprises a pressure sensor that is configured to detect contractions of a stomach in which reservoir **12** is deployed, contractions which are indicative of an event such as hunger or food ingestion. When such an event is detected, event detector **48** transmits the fact of detection to controller **40**.

Power storage unit **44** is a rechargeable battery functionally associated with components of device **10** that require power such as actuator **38** and especially with pressure generator **30** of spray head **14** through actuator **38** and power line **20**.

Power storage unit recharger **46** comprises a photovoltaic cell and other necessary components and is configured to convert near-infrared light impinging on the photovoltaic cell to electricity to recharge power storage unit **44**.

Controller **40** comprises a populated circuit board with appropriate electronic components and is functionally associated with timer **42** to constitute actuator **38** and connects between power storage unit **44** and pressure generator **30** of spray head **14** through power line **20**. Amongst other functions, controller **40** is configured so that upon receipt of a signal that an event is detected from event detector **48**, controller **40** allows power to pass from power storage unit **44** to pressure generator **30** of spray head **14** for a specified time with reference to timer **42**.

Casing **50** is substantially impervious to conditions in the stomach and comprises a two-part welded shell of a fluorocarbon polymer that is substantially transparent to light having near-infrared wavelengths that is converted by power storage unit recharger **46** to electricity.

Feeder tube **18** provides fluid communication between composition chamber **26** of reservoir **12** and spray head **14**. Feeder tube **16** is substantially a hollow tube of a material resistant to the condition of the gastrointestinal tract (e.g., a fluorocarbon polymer such as polytetrafluoroethylene). The length of feeder tube **16** is such that, when reservoir **12** is deployed in a stomach, spray head **14** lays in and is thus deployed in the descending portion of the duodenum.

Power line **20** is substantially a cable that provides power from control unit **18** to pressure generator **30**.

A method applied for the treatment of an eating disorder (e.g., obesity, bulimia, eating disorders, overeating, diabetes-related obesity, metabolic syndrome) will be described with reference to device **10,** as described above.

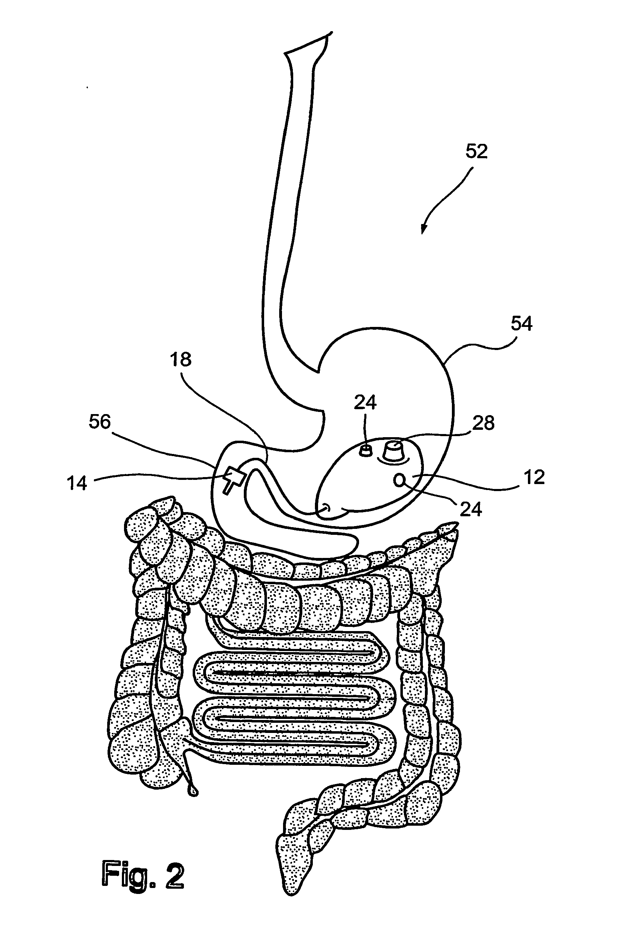

In Figure 2, device **10** is depicted deployed in a gastrointestinal tract **52** of a person suffering from an eating disorder, where reservoir **12,** deployed in stomach **54** is in fluid communication with spray head **14** located in the descending portion of duodenum **56** through feeder pipe **18**.

Prior to the depicted in Figure 2, device **10,** when space-filling chambers **22** and composition chamber **26** are empty, is placed in stomach **54** with the help of a gastroscope. Spray head **14** is passed through the pyloric sphincter and deployed in duodenum **56**. Using a gastroscope, reservoir **12** is deployed in stomach **54** by pumping saline solution into space-filling chambers **22** through ports **24** to a desired extent (e.g., 150 ml in each chamber **22)** which is dependent, in part on the size of stomach **54** and in part to ensure that spray head **14** remains in duodenum **56**.

Using a gastroscope, composition chamber **26** is charged (e.g., 100 ml) through port **28** with a sprayable pharmaceutical composition comprising an active agent suitable for treating an eating disorder. As discussed in the PCT patent application published as WO 2006/035446 of the Applicant, suitable active agents include but are not limited to satiety agents (e.g., CCK, CCK receptor agonists, PYYs, GLP-1, and oxyntomudulin and analogs thereof and derivatives thereof) and anti-food absorption drugs such as lipase inhibitors. Significantly, suitable active agents include peptides and hormones that are challenging for gastrointestinal administration.

Once deployed, device **10** is configured to automatically administer a dose of the active agent to the subject, when necessary, by spraying the sprayable pharmaceutical composition through sprayer **34** of spray head **14** at the luminal wall of duodenum **56**.

The subject in which device **10** is deployed goes about life in the usual way. When the subject becomes hungry or begins to ingest food, stomach **54** begins to contract, a physiological change that is automatically detected by event detector **48.** The fact of detection of the event is transmitted by event detector **48** to controller **40** of actuator **38.**

Controller **40** triggers pressure generator **30** by allowing power from power storage unit **44** to pass to pressure generator **30** for a specified period of time as determined by timer **42.** The dose of active agent administered is determined by the specified period of time.

Pressure generator **30** pumps sprayable composition from reservoir **26** through feeder pipe **18** into sprayer **34.** The pressure at which pressure generator **30** pumps the sprayable composition into sprayer **34** forces the sprayable composition out through nozzles **32** at the luminal wall of duodenum **56.** The active agent in the sprayable composition interacts with satiety chemoreceptors found on the luminal wall of duodenum **56,** leading to a feeling of satiety in the subject. The feeling of satiety causes the person to eat less, thus treating the eating disorder.

It is important to note that reservoir **12** of device **10** performs at least one additional function in the framework of treating a person suffering from an eating disorder. As is clear to one skilled in the art, reservoir **12** has a stomach space-filling function in the manner of intragastric balloons known in the art for treating eating disorders to provide a feeling of fullness. In some embodiments, the magnitude of this effect is varied by adding or removing fluid from space-filling reservoirs **24** through ports **24.**

Periodically, for example at fixed intervals determined by a caregiver, composition reservoir **26** is recharged with sprayable composition through port **28** using a suitably-configured gastroscope. When recharging, the nature of the composition is optionally changed, e.g., by changing the concentration of the active agent in the composition or changing the identity of the active agent.

Periodically, for example at fixed intervals determined by a caregiver, power storage unit **44** is recharged. An gastroscope bearing a suitable source of near-infrared light is placed to illuminate the photoelectric cell of power storage unit recharger **46** which converts the light to electricity and recharges power storage unit **44.**

In device **10** of the present invention depicted in Figures 1, sprayer **14** is configured to be deployed in the duodenum, and in Figure 2 sprayer **14** is deployed in duodenum **56,** as the duodenum is the preferred location for administration of the satiety agents which device **10** is designed to administer. In some cases, a sprayer of the present invention may be deployed elsewhere, for example in the esophagus, the stomach, the antrum, antral sphincter, the fundus, the pylorus, the small intestine, the jejunum, the ileum, the large intestine, the caecum, the vermiform appendix, the colon, the ascending colon, the transverse colon, the descending colon, the sigmoid flexure or the rectum. Similarly, in some embodiments of the device of the present invention, a sprayer of the present invention is configured to be deployed elsewhere, for example in the esophagus, the stomach, the antrum, antral sphincter, the fundus, the pylorus, the small intestine, the jejunum, the ileum, the large intestine, the caecum, the vermiform appendix, the colon, the ascending colon, the transverse colon, the descending colon, the sigmoid flexure or the rectum. The specific location where a sprayer is deployed or is configured to be deployed is chosen as the preferred, the ideal or otherwise advantageous place for administration of the active agent, for example where the active agent is preferentially absorbed into the body by the gastrointestinal tract or where chemoreceptors that are affected by the active agent are located.

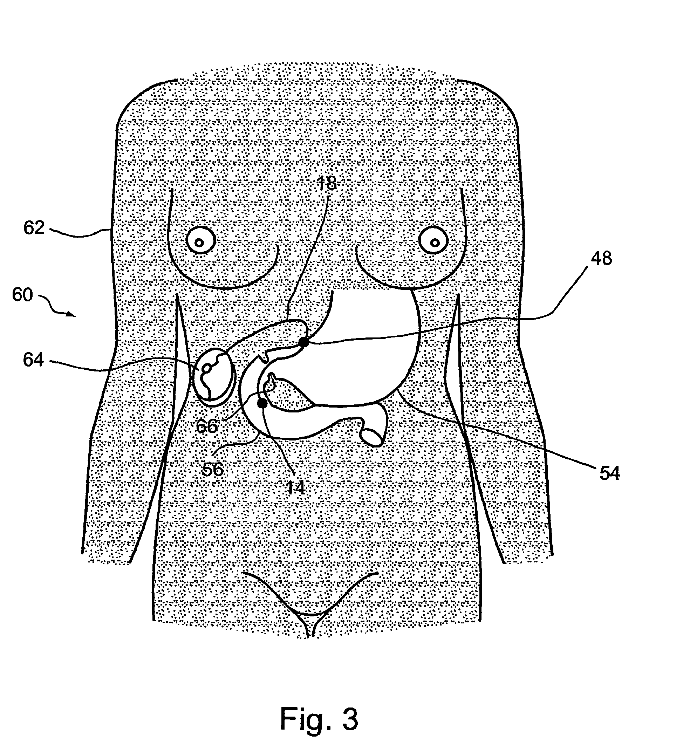

An additional embodiment of a device of the present invention configured for administration of satiety agents to the duodenum in accordance with the teachings of the present invention, device **60,** is depicted in Figure 3 deployed within a body of a subject **62.**

Device **60** comprises a control unit **64** (having dimensions of roughly between 4 cm and 10 cm long, between 3 cm and 6 cm wide and between 0.5 cm and 2 cm deep) that comprises a casing in which components including a composition reservoir, a power supply unit, a pressure generator (to dispense a dose of composition held in the composition reservoir) and an actuator (e.g., a controller associated with a timer). The pressure generator in control unit **64** is in fluid communication with a spray head **14** through feeder tube **18,** spray head **14** being deployed in the duodenum **56** of subject **62.** Event detector **48** (for example, similar to a gastric activity detector implemented in the Tantalus™ System of Metacure NV, MetaCure N.V., Curacao, Netherlands Antilles) is physically associated with feeder tube **18** and is functionally associated with the actuator in control unit **64** by a wire running from event detector **48** to control unit **64.**

Device **60** is substantially similar to device **10** discussed hereinabove with a number of notable differences.

A notable difference between device **10** and device **60,** as seen in Figure 3, is that device **60** is not simply deployed in the gastrointestinal tract of subject **62** but rather components thereof (control unit **64**) are implanted subcutaneously in the body of subject **62.**

Another notable difference is that unlike in device **10,** in device **60** a pressure generator (a pump) is physically located with control unit **64** and not with spray head **14.** The sprayer of spray head **14** of device **60** is simply a plugged tube with a plurality of nozzles arrayed about the axis of the tube and configured to direct a spray outwards.

Another notable difference is that, unlike in device **10,** event detector **48** is remote from control unit **64** and is implanted inside the wall of stomach **54,** allowing event detector **48** to detect and monitor gastric neural and muscle activity indicative of hunger or food ingestion. Event detector **48** serves an additional function, defining a passage through which feeder tube **18** passes through the wall of stomach **54,** but preventing the passage of gastric fluids from stomach **54** into the abdomen.

The use of device **60** is substantially similar to the use of device **10** as described above and is clear to one of average skill in the art upon perusal of the description and the Figures.

In some embodiments, deployment of a device **60** of the present invention requires a number of steps, performed in any convenient order as determined by a health care professional such as a surgeon. For example, control unit **64,** feeder tube **18,** event detector **48** and spray head **14** are introduced into the abdominal wall and implanted subcutaneously. Using a laparoscope, feeder tube **18,** event detector **48** and spray head **14** are threaded through the wall of stomach **54** and event detector **48** implanted across the wall of stomach **54.** Using an intraluminal endoscope, feeder tube **18** is placed so that spray head **14** is deployed in duodenum **56** through pyloric sphincter **66.** The reservoir in control unit **64** is charged (and when required, recharged) with a sprayable pharmaceutical composition comprising an active agent with the help of a transcutaneous needle as in known in the field of subcutaneously implanted devices including reservoirs (see for example the U.S. Patent Application published as US 2002/0087113).

As described above for device **10,** in some embodiments where a subject is treated for eating disorders, when subject **62** is hungry, sees feed or begins to consume food, event detector **48** detect physiological changes (such as electrical and mechanical activity of the stomach) indicative of the need for administering a sprayable pharmaceutical composition held in the reservoir, and transmits the fact of this need to the actuator in control unit **64.** The actuator activates the pump which forces sprayable composition from the sprayable composition reservoir in control unit **64,** through feeding tube **18** out through the nozzles of sprayer head **14** as a spray against the luminal walls of duodenum **56** thereby achieving a desired effect.

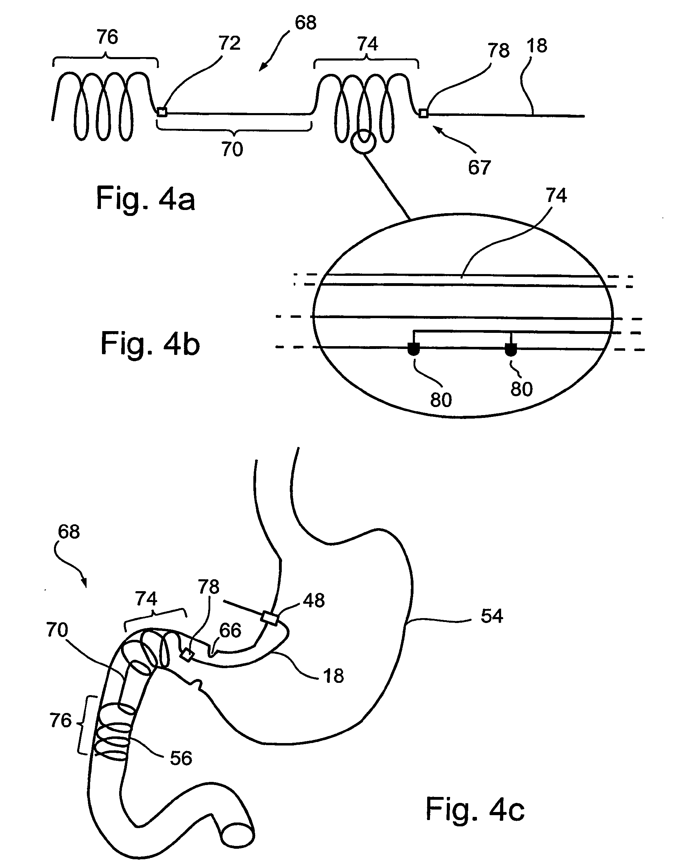

Depicted in Figures 4A, 4B and 4C is an embodiment of a spray head **68** of the present invention, substantially the distal end of a feeder tube **18.** In Figure 4A, spray head **68** is shown outside of the body of a subject. An enlarged cross section of the wall of spray head **68** is depicted in Figure 4B. Spray head **68** is shown deployed in a duodenum **56** in Figure 4C.

In spray head **68,** sprayer **70** is substantially a straight length of tube delimited by an intraluminal plug **72** in fluid communication with feeder tube **18** that is flanked between a proximal anchoring section **74** and a distal anchoring section **76.** Through the walls of the section of tube constituting sprayer **70** are a plurality of perforations arrayed about the axis of sprayer **70** constituting nozzles.

Spray head **68** is a flexible tube having an internal diameter of 2 mm and an outer diameter of 4 mm sections which are preshaped to assume a coiled shape to define proximal anchoring section **74** and distal anchoring section **76.** To the proximal end of proximal anchoring section **74** is attached a marker **78** that is both radiopoaque and ultrasound opaque. Protruding from the outside facing walls of proximal anchoring section **74** are a plurality of gold electrodes **80** (distanced between 1 and 10 cm one from the other) that are in electrical communication with a controller (not depicted).

For deployment, a flexible but straight wire guide is inserted into the lumen of feeder tube **18,** forcing feeder tube **18** to adopt a straight shape. Feeder tube **18** including spray head **68** is placed into the body (e.g., transcutaneously or through the mouth) and maneuvered past pyloric sphincter **66** into duodenum **56.** Once marker **78** is just past pyloric sphincter **66,** the wire guide is gradually withdrawn, allowing first distal anchoring section **76** and subsequently proximal anchoring section **74** to adopt a coil shape that presses against the luminal walls of duodenum **56.** In such a way, anchoring sections **74** and **76** both position, anchor and maintain sprayer **70** in a stretched-out configuration suspended substantially parallel with the luminal walls of duodenum **56** at a distance therefrom allowing efficient spray formation and spray coverage through the nozzles of sprayer **70.** Further, proximal anchoring section **74** prevents spray head **68** from being pulled out through pyloric sphincter **66** into stomach **54.** Further, electrodes **80** are pressed against the luminal wall of duodenum **56.** Intraluminal plug **72,** e.g., a partially expandable stainless steel plug, is anchored in the appropriate location inside feeder tube **18.**

The use of a sprayer **70** of a spray head **68** is, in analogy to the described above, clear to one skilled in the art upon perusal of the description herein. When needed, a pharmaceutical composition is sprayed through the nozzles of sprayer **70** against the luminal walls of duodenum **56.** In addition, when needed, electricity is passed through electrodes **80** under control of a functionally associated controller to stimulate nerves in the duodenal luminal wall, for example to give the perception of satiety. Thus, spray head **68** is configured to act through two modes: stimulation of nerves with the help of electrodes **80** and administration of an active agent through nozzles of sprayer **70.**

In some embodiments of spray head **68** especially configured for treating eating disorders, one or both of anchoring sections **74** and **76** are configured to press outwards with a substantial force so as to activate satiety mechanoreceptors. In such embodiments, a spray head **68** is configured to act through three modes: stimulation of nerves with the help of electrodes **80,** administration of an active agent through nozzles of sprayer **70** and stimulation of mechanoreceptors by applying an outwards force to the luminal walls of a duodenum.

In some embodiments of a device of the present invention such as device **10** depicted in Figures 1 and Figure 2 or device **60** depicted in Figure 3, a sprayer such as associated with spray head **14** is deployed at the specific location where administration of the sprayable composition is desired, and remains deployed at that specific location by a combination of the length of feeder tube **18** and the fact that the bulky reservoir **12** remains in place in stomach **54** (in the case of device **10)** or that the event detector **48** is fixed in place in the wall of stomach **54** (in the case of device **60).**

In some embodiments, a device of the present invention comprises one or more anchors, generally attached to or in the proximity of the sprayer for anchoring a deployed sprayer in place in proximity of the specific location. Anchors suitable for anchoring objects, such as a sprayer of an embodiment of a device of the present invention, in the gastrointestinal tract are well known in the art, see for example the PCT patent application published as WO2006/111961. As is known to one skilled in the art, implanted devices such as anchor are preferably implanted tension-free to prevent pain, tearing of surrounding tissue, migration and/or release of the anchor. Thus, it is preferred that an anchor anchoring a sprayer of a device of the present invention be tension free, for example, comprises a loose suture or loose stapling.

In Figures 1, 2 and 3, a spray head **14** may make incidental contact with luminal walls of a gastrointestinal tract in which deployed. In Figures 4, spray head **68** which is substantially the distal end of feeder tube **18** is configured, by coiling to position and anchor sprayer **68** suspended inside the lumen of the gastrointestinal tract, far from contact with a luminal wall.

In some embodiments are generally configured to provide a spray 360° around the axis of the respective sprayer.

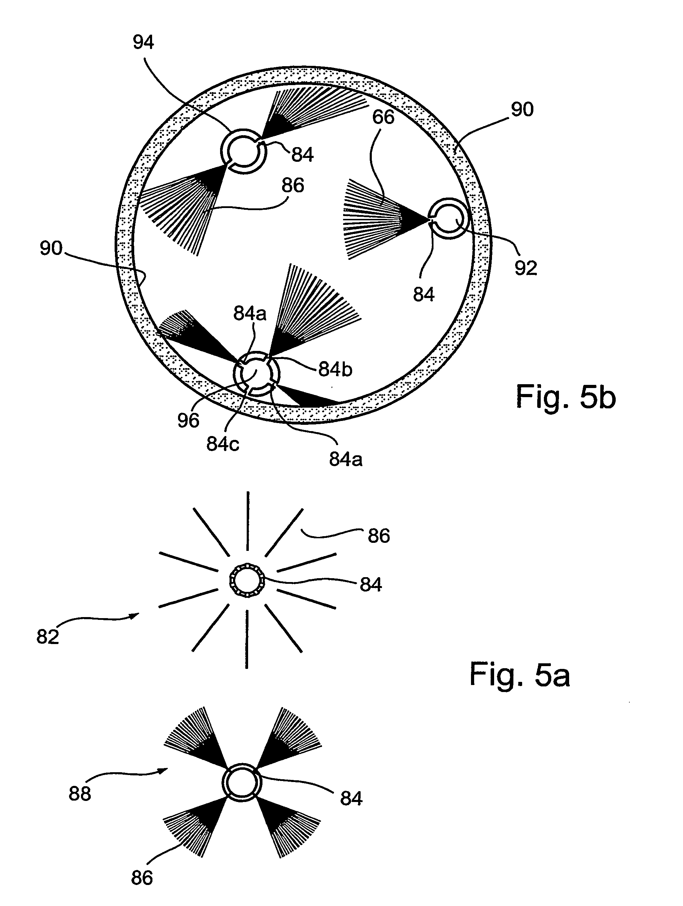

In Figure 5A are depicted two sprayers, in cross section. Sprayer **82** includes a plurality of rows of ten nozzles **84** (one such row depicted in the cross section depicted in Figure 5A), each row arranged about the circumference of sprayer **82** and each nozzle **84** configured to produce a narrow spray (substantially a stream) **86.**

Sprayer **88** depicted in Figure 5B includes a plurality of rows of four nozzles **84** (one such row depicted in Figure 5B), each row arranged about the circumference of sprayer **88** and each nozzle **84** configured to produce a wide spray (approximately an arc of 60°) **86.**

In some embodiments, a portion of a sprayer contacts a luminal wall, for example as a result of configuration for anchoring or deploying. For example, in a non-depicted embodiment similar to spray head **68** depicted in Figures 4, a sprayer, substantially a plurality of nozzles is a part of one or both the anchoring sections **74** or **76** that are coiled and press against a luminal wall of a gastrointestinal tract. In such embodiments, it is generally preferred that the sprayer be configured to spray over a relatively large area of the luminal wall, for example by an arrangement of nozzles configured and arranged to spray at parts of the luminal wall both near and far from where the sprayer contacts the luminal wall.

For example, in Figure 5B, three sprayers **92, 94** and **96** are depicted pressed against a luminal wall **90** of a gastrointestinal tract, all in cross section.

In Figure 5B, the depicted cross section of sprayer **92** includes a single nozzle **84** with an arc of approximately 60° towards a portion of luminal wall **90** opposite the part of luminal wall **90** that sprayer **92** contacts.

In Figure 5B, the depicted cross section of sprayer **94** includes two nozzles **84** each with an arc of approximately 45° directed towards a portion of luminal wall **90** close to the part of luminal wall **90** that sprayer **94** contacts.

In Figure 5B, the depicted cross section of sprayer **96** includes four nozzles **84** each with an arc of approximately 45° directed so that two nozzles **84a** are directed towards a near portion of luminal wall **90,** one nozzle **84b** is directed towards a distant portion of luminal wall **90,** and one nozzle **84c** is blocked.

Generally, it is desired that a pharmaceutical composition administered in accordance with the teachings of the present invention be administered to a relatively large area of the luminal wall of the gastrointestinal tract. To this end, in some embodiments a sprayer of a device of the present invention is configured to spray a length of no less than 1 cm of a luminal wall in which the sprayer is deployed, for example by including a plurality of nozzles along a length of the sprayer or by providing nozzles having a relatively large arc with respect to the length of the gastrointestinal tract. In some embodiments the length is of no less than 2 cm, of no less than 3 cm and even of no less than 4 cm.

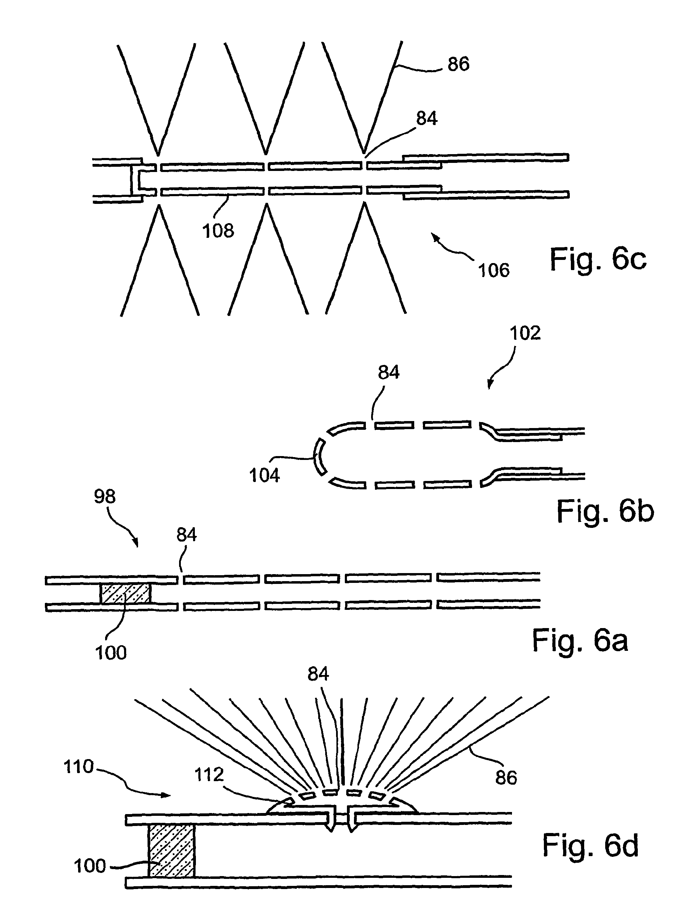

Some embodiments of sprayers suitable for implementing the teachings of the present invention are depicted in Figures 6A, 6B, 6C and 6D in side cross section.

In Figure 6A, sprayer **98** is substantially a portion of the distal end of a feeder tube wherein a plug **100** inside the feeder tube defines the distal end of sprayer **98** and nozzles **84** are substantially holes, perforations, slits and the like through the wall of the tube, substantially as described in Figure 3 and Figures 4.

In Figure 6B, sprayer **102** is substantially a perforated plug **104** capping the end of a feeder tube, comprising a plurality of nozzles **84.**

In Figure 6C, sprayer **106** is substantially a perforated closed-end tube **108** attached to end of a feeder tube, comprising a plurality of nozzles **84.**

In Figure 6D, sprayer **110** comprises one or more spray heads **112** including a plurality of nozzles **84** placed through the wall of the feeder tube.

It is seen that each of the sprayers depicted in Figures 6 is configured to spray a relatively significant length of a luminal wall in which deployed.

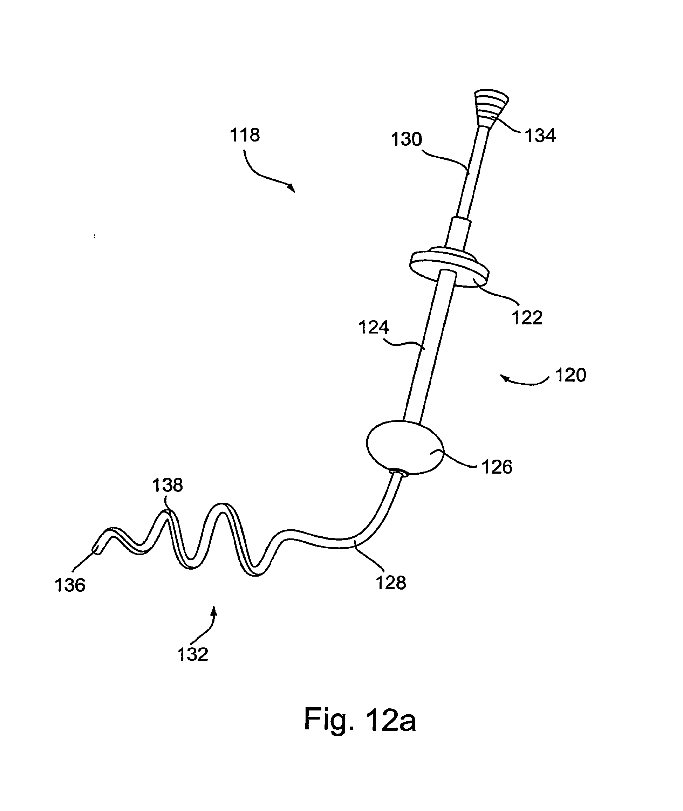

In Figures 12A-12E, an additional embodiment of a device useful for administering a sprayable composition to a luminal wall of the gastrointestinal tract (specifically the duodenum, specifically the superior portion of the duodenum) is depicted, device **118.** In Figure 12A, device **118** is depicted fully assembled and associated with a gastrostomy tube **120**

Gastrotomy tube **120** is a standard commercially available gastrostomy tube (e.g., MicTM-"G" available from Medical Innovations Corporation, a division of Ballard Medical Products, Draper, Utah, USA) including an external button **122,** a transabdominal tube **124** and an intragastric retainer balloon **126.**

In Figure 12A, it is seen that device **118** comprises a tubular body **128** having a proximal end **130** and a coiled distal end **132**. Tubular body **128** has structural features so as to be configured, amongst others, as a feeder tube, a sprayer and an anchor to maintain the sprayer portion properly positioned in the duodenum of a subject.

Proximal end **130** is provided with a connector **134** allowing connection of tubular body **128** to a pressure generator (such as a pump) and a composition reservoir.

Distal end **132** ending with distal tip **136** has a conical coil shape so as to have an increased-diameter relative to the rest of tubular body **128**. Coiled distal end **132** is configured to function as a sprayer: on the outer surface of distal end **132** is a slit **138** that functions as a spray orifice through which sprayable composition is forced out towards the luminal wall of a duodenum in which distal end **132** is deployed. As is discussed in detail below, slit **138** is configured to function as a valve, allowing a sprayable composition to spray outwards from tubular body **128** but substantially preventing passage of fluids into tubular body **128**.

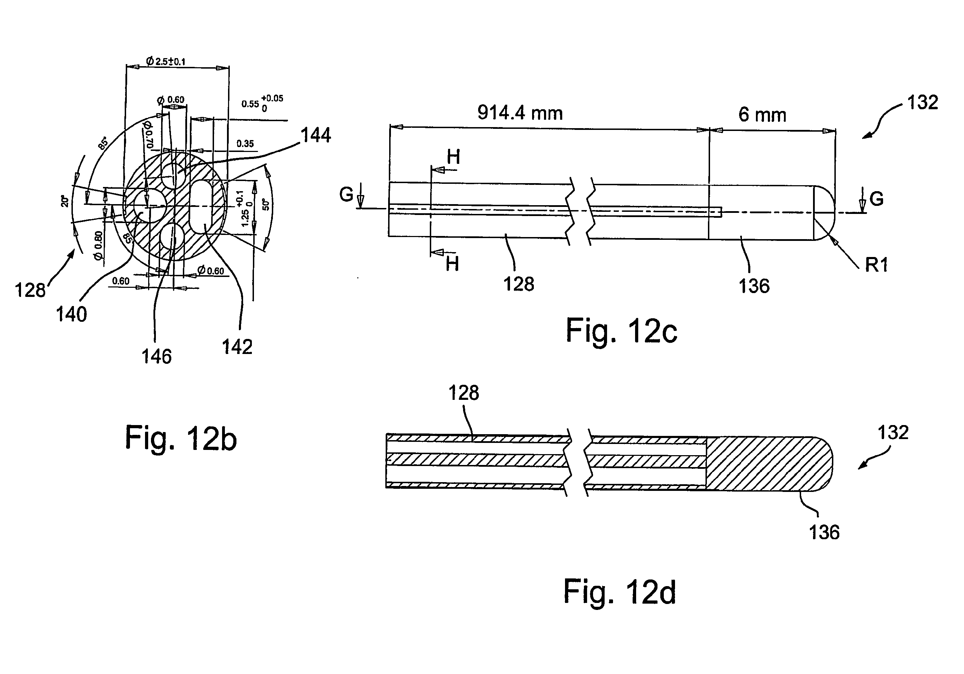

Tubular body **128** is substantially a 916 mm long by 2.5 mm diameter flexible tube of extruded Pebax 60 resin polymerized together with 20% barium sulfate. Passing coaxially through tubular body **128** are four parallel lumina. In Figure 12B, a radial cross-section near distal end **132** of tubular body **128**, is seen the arrangement of a 0.8 mm diameter active agent lumen **140**, a 0.55 mm wide by 1.25 mm high rounded-rectangle lumen **142** for accepting a Nitinol strip, and two 0.6 mm diameter round electrode guiding lumina **144** and **146**.

In Figures 12C and 12D, axial cross sections of tubular body **128** are depicted. It is seen that a 6 mm long rounded distal cap of soft polymerized Pebax 30D is secured as tip **136** of distal end **132** of tubular body **128**.

For assembly, a 914 mm long, 1 mm wide and 0.4 mm thick strip of Nitinol (not depicted) formed so that a distal end thereof adopts the desired shape of a 4 cm long conical coil having 3.5 loops is passed through rounded-rectangle lumen **142**. The Nitinol strip forces distal end **132** of tubular body **128** to adopt the conical coiled shape depicted in Figure 12A where active agent lumen **140** is on the outside of the coil. In such a way, coiled distal end **132** of tubular body **128** is configured to function as an expandable increased-diameter feature.

On the outer face of coiled distal end **132** of tubular body **128**, a sharp knife is used to make slice **138** coaxial to tubular body **128** through the wall of tubular body **128** to active agent lumen **140** forming an orifice. In such a way, coiled distal end **132** is configured as a sprayer, where slice **138** functions as a valve allowing a sprayable composition to be forced out as a spray, but preventing entry of liquids back into active agent lumen **140**, sees below.

In the art of gastrointestinal surgery, percutaneous endoscopic gastrostomy is used to endoscopically deploy a gastrostomy tube through the abdominal wall to provide a passage from the outside of the body into the stomach cavity.

In an embodiment for deploying device **118**, gastrostomy tube **120** is deployed in the usual way so that external button **122** contacts the skin of a subject and intragastric retainer balloon **126** is inflated inside the stomach cavity of the subject so that bodily tissue is clamped between external button **122** and intragastric retainer balloon **126** while transabdominal tube **124** defines a direct channel from outside the body to the stomach cavity.

Device **118** is threaded through a delivery tube (not depicted, but e.g., 3 mm inner diameter, 4 mm outer diameter braided stainless steel flexible tube lined with polytetrafluoroethylene and covered with a Pebax® polymer sleeve) forcing coiled distal end **132** into a straight conformation. While encased in the delivery tube, device **118** is threaded, distal tip **136** first, through transabdominal tube **124** of gastrostomy tube **120** into the cavity of a stomach of a subject. Under guidance of and with the help of a gastroscope, distal tip **136** is guided through the pyloric sphincter and into the duodenum. The delivery tube is carefully withdrawn while device **118** is pushed forward. As distal end **132** emerges from the delivery tube, distal end **132** expands into the coiled conformation. Ultimately, distal end **132** is completely coiled and the delivery tube entirely withdrawn from gastrostomy tube **120**. A retaining clip **148** is secured around tubular body **128** and against the outer side of external button **122**, to act together with tubular body **128** as an anchor so that distal end **132** is maintained in the superior portion of the duodenum.

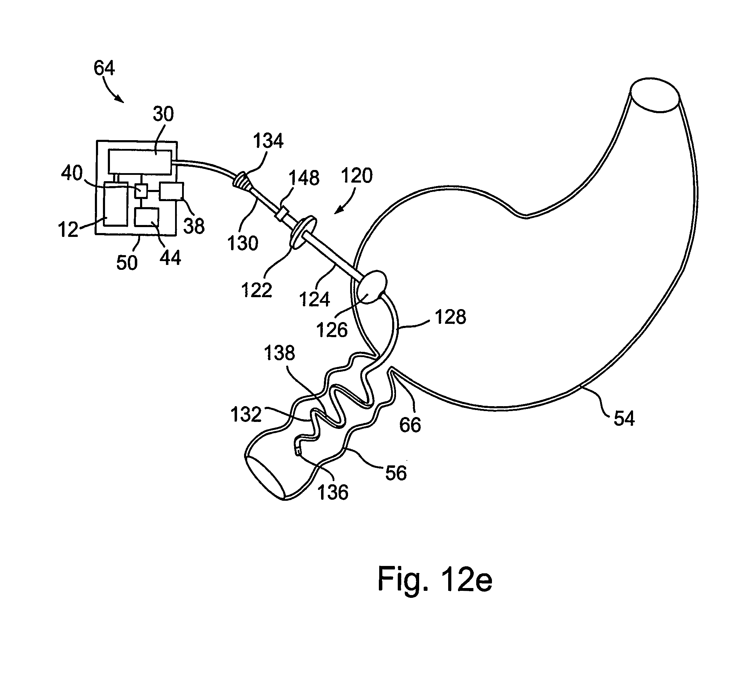

In Figure 12E, device **118** is depicted properly deployed in the gastrointestinal tract of a subject, where the abdominal wall and other abdominal tissue are not depicted. In Figure 12E is seen how external button **122** contacts the skin and inflated intragastric retainer balloon **126** contacts the inner surface of stomach **54** so as to clamp bodily tissue therebetween so that transabdominal tube **124** defines a direct channel from outside the body to the cavity of a stomach **54**. Connector **134** and proximal end **130** of tubular body **128** are located outside the body of the subject. Tubular body **128** passes through pyloric sphincter **66** while coiled distal end **132** of tubular body **128** is located in the superior portion of duodenum **56**.

Coiled distal end **132** of tubular body **128** functions as a sprayer and also as an increased-diameter portion so as to prevent distal end **132** from moving outwards through pyloric sphincter **66**. Clip **148** together with the length of tubular body **128** act as an anchor to maintain coiled distal end **132** in the superior portion of duodenum **56** about 1 cm from pyloric sphincter **66**.

For use, a control unit **64** is mated to connector **134**. Control unit **64** includes a pressure generator **30**, a composition reservoir **12**, an actuator **38** (a manually operable switch), a controller **40** and a power storage unit **44** and is configured to be deployed outside of the body of the subject. Control unit **64** is similar to the control unit of the DuoDopa® device (Solvay Pharmaceuticals GmbH, Hannover, Germany).

When actuator **38** is triggered, controller **40** actuates pressure generator **30** to pump sprayable composition (e.g., a pharmaceutical composition including an active agent) from composition reservoir **12,** past connector **134,** into active agent lumen **140** of tubular body **128.** The pressure forces the sprayable composition through slit **138** as an outwardly oriented sheet-like spray, administering the composition in accordance with embodiments of the invention. After sufficient time has passed for a desired dose to have been administered, controller **40** stops pressure generator **30** so that composition is no longer forced through slit **138** and the pressure in active agent lumen **140** is reduced. When the pressure is reduced, the elasticity of the walls of body **128** forces slit **138** closed, preventing entry of materials into active agent lumen **140.**

In device **118,** a prior art gastrostomy tube **120** is used to define a passage through which tubular body **128** of device **118** passes into the body of the subject. In some embodiments, a device body such as tubular body **128** is fashioned having features (e.g., integrally formed with or attached to) of a gastrostomy tube **120** such as external button **122** and intragastric retainer balloon **126** rendering a separate transabdominal tube **124** unnecessary. In some embodiments, a different type of gastrostomy tube or functionally equivalent component is used.

In some embodiments, instead of a control unit such as **54** located outside of the body of the subject, a control unit is configured to be implantable inside the body (for example subcutaneously, in the abdominal cavity) or inside the gastrointestinal tract, as discussed hererinabove and hereinbelow.

In some embodiments of a device of the present invention such as device **10** depicted in Figures 1 and Figure 2, a reservoir such as reservoir **12** is deployed in the stomach. Deploying in the stomach is simple as the stomach is relatively large and flexible allowing a relatively large reservoir to be deployed therein with no ill effects. The stomach is easily accessible through the esophagus with a gastroscope, allowing relatively simple deployment and maintenance (such as recharging of a composition reservoir or a power supply unit) of the device. Further, in some embodiments contractions of the muscular stomach assist in driving sprayable composition from a composition reservoir to the sprayer. Further, as discussed above for device **10**, in some embodiments a reservoir deployed in the stomach acts as an intragastric space-filling balloon, a property useful as an adjunct when treating certain ailments.

Despite the advantages of deploying the reservoir of a device of the present invention in the stomach, in some embodiments a reservoir is deployed elsewhere in the gastrointestinal tract, for example closer to the specific location where the sprayable composition is to be sprayed. Although in some cases a reservoir is deployed anywhere in the gastrointestinal tract, preferred locations for deploying a reservoir of a device of the present invention include the large intestine, the caecum, the vermiform appendix, the colon, the ascending colon, the transverse colon, the descending colon, the sigmoid flexure or the rectum.

In some embodiments of a device of the present invention such as device **60** depicted in Figure 3, a reservoir such as reservoir **12** is deployed and anchored subcutaneously. Subcutaneous implantation of composition holding reservoirs is well-known in the art and allows for simple recharging of the reservoir with a pharmaceutical composition including an active agent when necessary. Despite the advantages of deploying a device of the present invention subcutaneously, some embodiments include a reservoir deployed elsewhere in the body.

In some embodiments, an actuator is configured to actuate the pressure generator so as to dispense a specified dose of sprayable composition. In some embodiments such as device **10** depicted in Figures 1 and Figure 2 or device **60** depicted in Figure 3, the dispensed dose is a fixed dose determined by the time which actuator **38** comprising controller **40** and timer **42** trigger pressure generator **30** to produce a spray by providing power from power storage unit **44.** In some embodiments, a device of the present invention comprises a dosage adjusting mechanism functionally associated with the actuator and/or with the pressure generator that allows the dosage to be changed or adjusted while the device is deployed in the gastrointestinal tract. For example, in some embodiments a dosage adjustment mechanism is functionally associated with the actuator and functions by changing the length of time which an associated pressure generator is actuated thus changing the dose. For example, in some embodiments, a device of the present invention comprises a wireless receiver functionally associated with a controller and the controller is configured to accept commands to change the length of time that the pressure generator is actuated. For example, in some embodiments a dosage adjustment mechanism is functionally associated with the pressure generator and functions by changing the volume of a composition that is sprayed per unit time, for example by changing or adjusting the stroke volume of a piston or like device. In some embodiments, the dosage adjusting mechanism is mechanical and is performed endoscopically, for example by turning a stroke-volume adjusting screw.

As noted above, some embodiments of the present invention include an event detector functionally associated with an actuator so that as a result of detection of an event of significance for administration of the active agent, the event detector triggers the actuator to administer the active agent. In device **10** depicted in Figures 1 and Figure 2, event detector **48** is a pressure sensor configured to detect a gastric contraction event associated with hunger or food ingestion. In device **60** depicted in Figure 3, event detector **48** is an electrical activity sensor configured to detect an electrical activity event in the wall of stomach **54** associated with hunger or food ingestion. One skilled in the art, upon perusal of the disclosure herein, is able to select and modify any of the different event detectors and sensors known in the art to implement of the teachings of the present invention, for example the electrode-comprising detectors disclosed in the PCT patent application published as WO 2006/035446 of the Applicant, gastric activity detectors such implemented in the Tantalus™ System (Metacure NV, MetaCure N.V., Curacao, Netherlands Antilles), or event detectors described in the U.S. Patent Application published as US 2005/0096637, pressure sensors (e.g., Chronicle® Medtronic, Inc., Minneapolis, MN, USA), muscle activity sensors such as described in the U.S. Patent Application published as US 2004/0220633 or available from Delsys Inc. (Boston, MA, USA), pH sensors (e.g., Bravo®, Medtronic, Inc., Minneapolis, MN, USA).

In some embodiments, an event detector is in wired communication with the actuator. In some embodiments, an event detector is in wireless communication (e.g., radio frequency or near-infrared communication) with the actuator.

In device **10** depicted in Figures 1 and Figure 2 and in device **60** depicted in Figure 3, actuator **38** comprising controller **40** and timer **42** is configured to trigger pressure generator **14** to begin administering a dose of sprayable composition upon detection of an event by event detector **48**. In some embodiments, actuator **38** is functionally associated with a timer and is configured to begin administering a dose of sprayable composition a specified period of time after detection of an event as determined by the timer. In some embodiments, the specified period of time can be changed or adjusted while the device is deployed in the gastrointestinal tract. For example, in some embodiments, a device of the present invention comprises a wireless receiver functionally associated with a controller and the controller is configured to accept commands to change a delay between detection of an event and initiation of administration of the sprayable composition.

In some embodiments, administration of a composition is event-driven, that is subsequently to detection of an event that is of significance for administration of the active agent whether manually (by the subject or by a caregiver) or automatically (by an event detector such as event detector **48** of device **10** depicted in Figures 1 and Figure 2 or device **60** in Figure 3), a sprayable composition is administered in accordance with the teachings of the present invention. In some embodiments, administration of a composition is periodic and administration of a composition in accordance with the teachings of the present invention is initiated according to a periodic schedule, whether manually by the subject or care giver, or automatically with a device configured for such.

In some embodiments, a device of the present invention is configured for periodic administration of a composition by functionally associating an actuator with a timer, and the actuator is configured to periodically trigger the pressure generator with reference to the timer. In some embodiments, the administration protocol (how often a dose is administered) is specified. In some embodiments, the device is configured to allow the administration protocol to be changed or adjusted while the device is deployed in the gastrointestinal tract. For example, in some embodiments, a device of the present invention comprises a wireless receiver functionally associated with a controller and the controller is configured to accept commands to change the frequency or timing or other parameters of the administration protocol.

In device **10** depicted in Figures 1 and Figure 2 and device **60** depicted in Figure 3 pressure generator **30** comprises an electrical pump to spray a sprayable composition in accordance with the teachings of the present invention. Other suitable devices useful as pressure generators to implement the teachings of the present invention include such devices as spring-powered pressure generators, gas-pressure powered pressure generators (comprising, for example, a compressed gas reservoir and a valve) and syringes.

As described above, device **10** depicted in Figures 1 and in Figure 2 is provided with a power supply unit including power storage unit **44** (a battery) and power storage unit charger **46**. In some embodiments a device of the present invention is provided with a non-rechargeable power storage unit. In some embodiments, a power supply unit of a device of the present invention includes a power generation unit, e.g. a kinetic power generation unit, similar to the described in U.S. Patent No. 6,154,422 that converts motions (such as shaking, moving or jostling) of an object with which a kinetic power generation unit is associated to electrical power.

As described above, device **10** depicted in Figures 1 and Figure 2 and device **60** depicted in Figure 3 are configured to administer an active agent through a sprayer deployed in the gastrointestinal tract when necessary. In some embodiments, administration of an active agent when necessary is optionally accompanied by additional modes and methods of treatment that are used continuously, simultaneously with or in parallel with the administration of the active agent. For example, in analogy to the discussed in the PCT patent application published as WO 2006/035446 of the Applicant, in some embodiments of the invention useful for treating eating disorders, additional modes and methods of treatment include direct vagal nerve stimulation using an implanted electrode or other forms of gastric stimulation such as implemented in the Tantalus™ System (Metacure NV, MetaCure N.V., Curacao, Netherlands Antilles).

Described above are some embodiments of the present invention used to treat conditions relating to eating disorders such as obesity, where a satiety agent is administered by spraying directly to interact with chemoreceptors lining the duodenal lumen in response to detection of a physiological change (stomach contraction, stomach wall electrical activity) indicative of an event (hunger, food ingestion) of significance to the need of administration of the satiety agent. In some embodiments, the teachings of the present invention are used for treating other conditions.

In some embodiments, the teachings of the present invention are used to administer an active agent that does not affect chemoreceptors at or near the luminal walls but rather is absorbed through the luminal wall and into the blood stream.

For example, in an embodiment, the teachings of the present invention are used to treat subjects suffering from a cardiovascular pathology and specifically to administer an active agent when an event such as an anginal attack or arrhythmia is detected. For example, the electrical activity of the heart is monitored in real time using a pacemaker such as a Model 1298 Insignia (Guidant Corporation a part of Boston Scientific, Inc. Natick, MA, USA) provided with wireless transmitter to a device of the present invention provided with an actuator functionally associated with a wireless receiver.

When the actuator receives an indication that an anginal attack or arrhythmia has been detected, an anti-arrhythmic or anti-anginal material as an active agent is administered through a sprayer deployed in the ileum. The active agent is rapidly absorbed, giving an almost immediate effect in response to the anginal attack or the arrhythmia. Suitable active agents include adenosine or nitrates (e.g., isosorbide dinitrate, isosorbide mononitrate, and nitroglycerin).

For example, in some embodiments, the teachings of the present invention are used to treat subjects suffering from asthma and specifically to administer an active agent when an event such as an asthmatic attack is detected. For example, a person suffering from asthma is provided with a portable trigger (e.g., a wrist borne "panic button") provided with wireless transmitter to a device of the present invention provided with an actuator functionally associated with a wireless receiver.

When the actuator receives an indication that an asthma attack has been detected for example, the "panic button" has been pressed, an anti-asthma material as an active agent is administered through a sprayer deployed in the ileum. The active agent is rapidly absorbed, giving almost immediate relief from the asthma. Suitable active agents include systemic bronchial activators such as salbutamol.

For example, in some embodiments, the teachings of the present invention are used to treat subjects suffering from an allergy and specifically to administer an active agent when an event such as an allergic reaction, especially as systemic allergic reaction such as anaphylaxis is detected. For example, a person suffering from an allergy is provided with a portable trigger (e.g., a wrist borne "panic button") provided with wireless transmitter to a device of the present invention provided with an actuator functionally associated with a wireless receiver.

When the actuator receives an indication that an allergic reaction has been detected for example, the "panic button" has been pressed, an anti-allergic or anti-anaphylactic material as an active agent is administered through a sprayer deployed in the ileum. The active agent is rapidly absorbed, giving almost immediate relief from the allergic reactions. Suitable active agents include epinephrine.

In some embodiments, the teachings of the present invention are used to periodically administer an active agent for maintenance or prophylaxis rather than in response to detection of an event.

For example, in an embodiment, the teachings of the present invention are used to treat subjects suffering from inflammatory bowel disease and specifically to topically treat inflammation in the gastrointestinal tract by administering a sprayable composition including an anti-inflammatory such as acyl salicylic acid. In an embodiment of the invention, a reservoir of a device of the present invention is charged with a sprayable acyl salicylic acid composition and a timer of a control unit is functionally associated with an actuator to periodically trigger the actuator to administer one or more daily doses of the composition. The sprayer is deployed in the terminal ileum and preferably configured to spray directly at an inflamed region.

The use of a device of the present invention to implement the method described herein has many advantages over prior art oral administration including that patient compliance is absolute even for complex administration regimens so that dosage is carefully controlled and can occur multiple times over day, ensuring a relatively constant plasma concentration and where an active agent administered comprises multiple materials.

For example, in an embodiment, the teachings of the present invention are used to treat subjects suffering from an infection of helicobacter pylori and specifically to topically treat ulcers in the gastrointestinal tract (both gastric ulcers and duodenal ulcers) by administering a sprayable composition including an anti-ulcer active agent. In an embodiment of the invention, a reservoir of a device of the present invention is charged with a sprayable anti-ulcer composition and a timer of a control unit is functionally associated with an actuator to periodically trigger the actuator to administer one or more daily doses of the composition. The sprayer is deployed in the vicinity of ulcers and preferably to spray directly at an ulcer.

In some embodiments, a composition includes an antibiotic, for example Amoxicillin, Clarithromycin, Metronidazole or Tetracycline. However, it is known that treatment of a helicobacter pylori infection is best performed by administration of an active agent comprising two, three or even four different active materials.

In some embodiments, a composition includes two materials, such as an antibiotic and a proton pump inhibitor (PPI), e.g., Esomeprazole, Lansoprazole, Omeprazole, Patoprazole or Rabeprazole. Particularly suitable are combinations of Clarithromycin with a PPI.

In some embodiments, a composition includes three materials, such as Clarithromycin with Metronidazole and a PPI; Amoxicillin with Clarithromycin and a PPI; Amoxicillin with Metronidazole and a PPI; or Tetracycline with Metronidazole and a cytoprotective agent (e.g. Bismuth subsalicylate or preferably sucralfate).

In some embodiments, a composition includes four materials, such as a cytoprotective agent (e.g. sucralfate or preferably Bismuth subsalicylate) with Metronidazole and Tetracycline and a H2 Blocker (e.g., Cimetidine, Famotidine, Nizatidine and Ranitidine); a cytoprotective agent (e.g. sucralfate or preferably Bismuth subsalicylate) with Metronidazole and Amoxicillin and a H2 Blocker; a cytoprotective agent (e.g. sucralfate or preferably Bismuth subsalicylate) with Metronidazole and Tetracycline and a PPI and a cytoprotective agent (e.g. sucralfate or preferably Bismuth subsalicylate) with Metronidazole and Clarithromycin and a PPI.

It is expected that during the life of this patent many relevant detectors, active entities and useful physical components such as pumps will be developed and the scope of the patent is intended to include all such a priori.

Additional objects, advantages, and novel features of the present invention will become apparent to one ordinarily skilled in the art upon examination of the following examples, which are not intended to be limiting. Additionally, each of the various some embodiments and aspects of the present invention as delineated hereinabove and as claimed in the claims section below finds experimental support in the following examples.

## **EXAMPLES**

Reference is now made to the following examples which together with the above description illustrate the invention in a non limiting fashion.

A study of the feasibility of the teachings of the present invention was performed in the context of treating the obesity using CCK-8.

CCK-8 is an 8 amino acid long fully active peptide analogue of CCK (the enteric hormone cholecystokinin). CCK is secreted from duodenal cells in response to the presence of food in the duodenum. The primary effects of CCK secretion are the release of bile from the gallbladder into the gut lumen and increased pancreatic secretion. CCK is also an enteric satiety factor. It is known that intravenous administration of CCK causes termination of food consumption by delaying gastric emptying and inducing a feeling of satiety. It is known that the CCK-induced bile release from the gallbladder and increased pancreatic secretions are mediated through CCKa receptors located on vagal nerve endings in the duodenal wall. It has been hypothesized that administration of CCK or an analogue thereof to the duodenal wall would lead to activation of CCKa receptors on the duodenal wall, leading to a feeling of satiety and meal termination which would reduce the amount of food eaten.

Obese volunteer subjects having a BMI greater than 35 kg cm$^{-2}$ were selected. The subjects were all between 18 and 50 years old, had no history of heart disease (MI, arrhythmia), no history of kidney disease or proteinurea (serum creatinin < 1.5 mg dl$^{-1}$), no history of peptic ulcers, intestinal disease, intestinal surgery, pancreatitis, gall stones or liver disease (SGOT, SGPT, Alk Phos < X3), no endocrine dysfunction, no diabetes mellitus, no active medical disease except hypertension, were taking no medication except thyroid supplements and medications for controlling blood pressure and blood lipids.

The subjects were fitted with intragastric balloons and various types of nasoduodenal tubes deployed in the duodenum. No less than two weeks after fitting with the intragastric balloon, those subjects that showed no complications resulting from the nasoduodenal tube (e.g., vomiting) were considered as having enrolled in the study, that day being designated as the enrollment day. In a first three week period the subjects were acclimatized to the study, learning to eat with the nasoduodenal tube and becoming familiar with the study methods. Various parameters of CCK-8 administration were tested including frequency, dose, the nature of the pharmaceutical composition including CCK-8 and the nature of the nasoduodenal tube.. It was unexpectedly found that a fast and forceful introduction of CCK-8 containing composition through nasoduodenal tubes configured with holes that lead to spraying of the composition towards the luminal walls of the duodenum was, all things being equal, significantly more efficient in eliciting a response attributable to CCK-8 absorption than a lower rate introduction where the composition flowed or dripped into the duodenum.

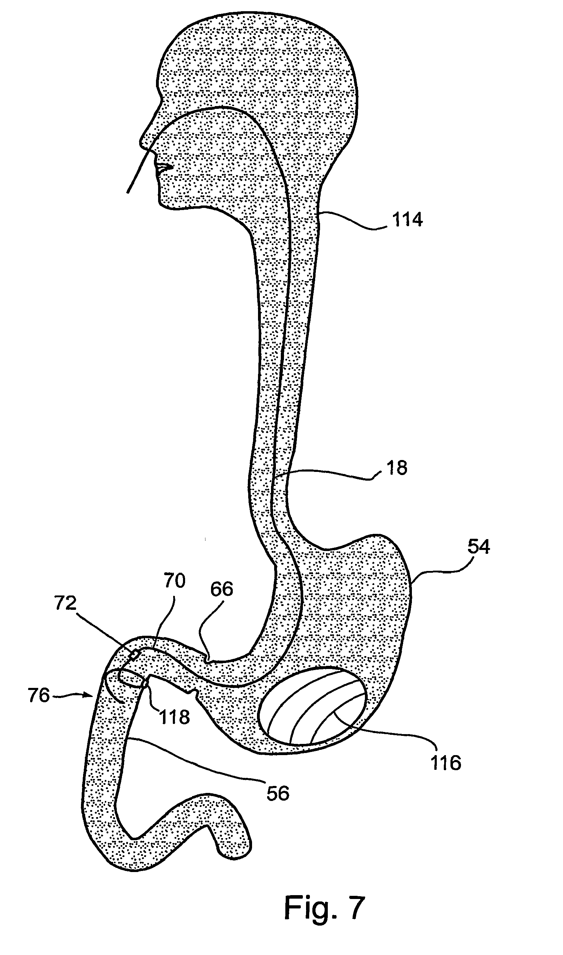

An administration device (schematically depicted in Figure 7) in accordance with the teachings of the present invention was made. A length of "pig tail" nasobiliary tube (Cook Nasal Biliary Drainage Sets, ENBD-6-Liguory, GPN G21725) was taken as a feeder tube **18**. Approximately 40 cm from the distal end, tube **18** was blocked with a stainless steel 316 LVM plug **72**. Tube **18** was perforated (on the proximal side of and close to plug **72**) with three rows of three nozzles each constituting a sprayer **70**, each row parallel to the tube axis and 120° from the other rows, each nozzle substantially a 0.5 mm diameter perforation in the tube wall directed perpendicularly outwards from the axis of tube **18**.

The subjects **114** were fitted with an intragastric balloon **116** followed by endoscopic placement of the administration device so that sprayer **70** was situated in the superior portion of the duodenum **56**. Placement of a sprayer **70** in the proper location in duodenum **56** was assisted by using plug **72** as a marker apparent in fluoroscopy and sonoscopy. Sprayer **70** was maintained in place by the 40 cm distal pig-tail that extended further into duodenum **56** as a distal anchoring section **76**, by friction of the curly "pig tail" structure with the luminal walls of duodenum **56**, and by anchoring with the use of a clip **118** (using a Resolution™ Clip Clipping Device REF 2261, Boston Scientific) to the duodenum wall.

Doses of approximately 6 ml of a sprayable composition including an amount of CCK-8 (Clinalpha Ltd.) mixed with a pharmaceutically acceptable carrier such as saline were administered over a 10 second period using a syringe attached to the proximal end of the feeding tube (intraduodenal bolus injection). The relatively high rate of administration ensured that the composition was sprayed (rather than dripped) out through the nozzles of the sprayer at the luminal wall of the duodenum where the CCK-8 of the composition interacted with receptors on the luminal wall to induce a feeling of satiety.

Effect of CCK-8 administration on gallbladder contraction

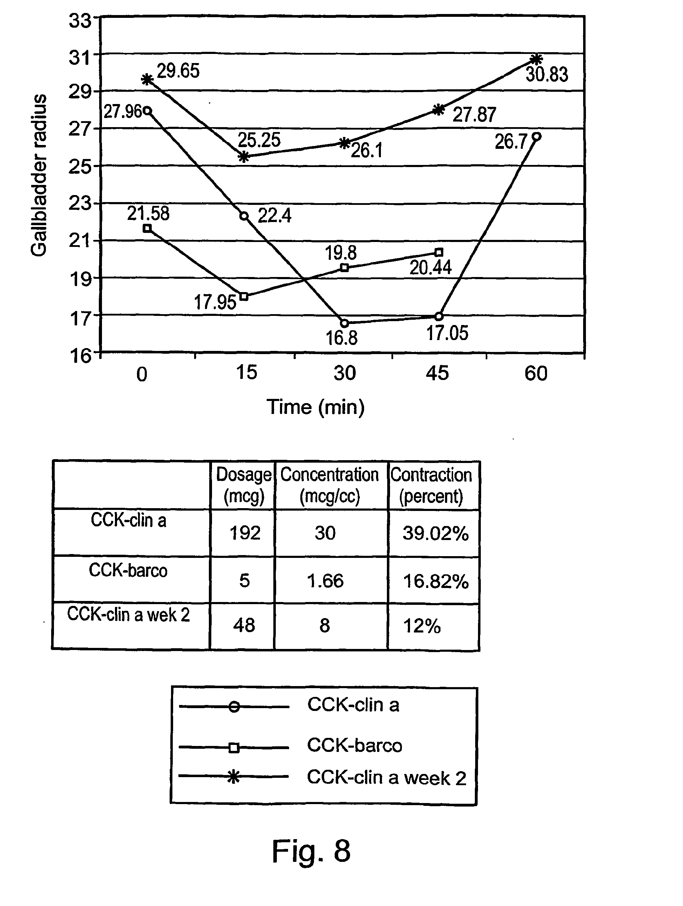

The size of the filled gallbladder of two patients in a fasting state was measured using ultrasonography. Fifteen minutes later, a dose of the sprayable composition was administered. To subject A was administered a dose of 192 μg CCK-8 (Clinalpha, Darmstadt, Germany) in a 6.4 ml volume. To subject B was administered a dose of 5 μg CCK-8 (Braco SpA, Milano, Italy) in a 3 ml volume and after two weeks 48 μg CCK-8 (Clinalpha, Darmstadt, Germany) in 6 ml. Ultrasound imaging and recording of the gallbladder was performed every 15 minutes to determine its ejection fraction. The examination was performed at 15, 30, 45 and 60 minutes or until substantial expansion of the gallbladder occurred. All images and recordings were acquired from the same angle and by the same technician using a Hitachi 8500 ultrasound device with a probe. The gallbladder volume was calculated using the dedicated software of the ultrasound device. The ejection fraction was calculated by dividing the volume of the contracted bladder by its volume at baseline (extended bladder). The results are presented in Table I and in Figure 8.

TABLE I: Effect of administration of CCK-8 in accordance with the teachings of the present invention on gallbladder contraction

| Subject | Dose (μg) | Concentration (μg ml$^{-1}$) | Contraction (%) |
|---|---|---|---|
| A | 192 | 30 | 39.02 |
| B (first) | 5 | 1.7 | 16.82 |
| B (second) | 48 | 8 | 12 |

Intraduodenal administration of CCK-8 in accordance with the teachings of the present invention led to a clear-cut and substantial contraction of the gallbladder. It was seen that when administered in accordance with the teachings of the present invention, CCK-8 led to up to about 39% gallbladder contraction, substantially equal to the $46.4 \pm 19.5\%$ contraction of the gallbladder known in the art for gallbladder contraction caused by 4 μg intravenously administered CCK-8. Although the amount of CCK-8 administered was higher (192 μg as opposed to 4 μg), it must be remembered that the results were obtained in preliminary experiments. Further, it must be remembered that the non-invasive administration of an active agent in accordance with the teachings of the present invention is preferable to invasive intravenous administration, even if a larger amount of active agent must be used.

Effect of CCK-8 administration on caloric consumption, food consumption and food taken to the plate

Six subjects from amongst the enrollees were selected for participation in the study, Table II.

TABLE II: Characteristics of subjects participating in study

| | Age | sex | Height [cm] | Wight [kg] | BMI |
|---|---|---|---|---|---|
| 1 | 43 | M | 182 | 144.5 | 44.6 |
| 2 | 47 | F | 162 | 150.5 | 58.8 |
| 3 | 48 | M | 166 | 117.0 | 40.5 |
| 4 | 34 | M | 155 | 117.0 | 49.0 |
| 5 | 34 | M | 176 | 122.4 | 39.5 |
| 6 | 42 | F | 164 | 128.0 | 47.6 |

No less than four weeks from the enrollment day of the subjects, an administration device as described above was inserted endoscopically to the six subjects. Sprayer location was evaluated twice a week by fluoroscopy. Reintroduction was performed when the sprayer was not properly located.

The six subjects stayed in the recovery department of a hotel associated with a medical center. Meals were served in a private room at the hotel restaurant in pleasant surroundings. The amount of food on a plate was weighed at the beginning and end of a meal to give an accurate estimate of food taken and consumed. Video recordings of the subjects were taken of all subjects throughout the meals.

Food weighing was performed with the plate twice before and after every meal, where one type of plate was used for breakfast and dinner and a different type of plate used for lunch. Additional weighing was performed whenever a subject took an extra portion. Drinks were calculated by glass rather than weighed.

Caloric intake was calculated in accordance with the following values: (calories per 100 gram): bread (250), cheese (100), vegetables/salad (40), egg (133), tuna (200), yoghurt (60), vegetable soup (67), meat/fish (170), boiled vegetable (40), rice/mashed potatoes (267) and fruit dessert (67).

The six subjects were served three meals a day. Breakfast and dinner were served as buffets in which subjects could choose as much food as desired. The buffets included bread, vegetables, vegetable salad, several sorts of low fat cheeses, egg, tinned tuna, yoghurt and juice or water. Lunch was a la carte and included soup and/or salad as a first course, a main course of meat or fish, rice or mashed potatoes, steamed vegetable, a fruit dessert and water or juice.

Two hours before a meal, saline as a placebo and a sprayable composition was prepared including CCK-8 as an active ingredient at the hospital pharmacy. The composition and placebo were placed in identical vials labeled only with the subject's name. The subject and the treating health-care professional were not aware which subject received a placebo and which the composition.

Five minutes prior to each meal, depending on the subject a placebo, a composition of 48.4 $\mu$g in 6 ml or of 96.8 $\mu$g in 9 ml was administered over an approximately 10 second period using a syringe attached to the proximal end of the feeding tube. The relatively high rate of administration ensured that the composition was sprayed (rather than dripped) out through the nozzles of the sprayer at the luminal wall of the duodenum where the CCK-8 of the composition interacted with receptors on the luminal wall to induce a feeling of satiety.

The weight and composition of food taken and consumed was recorded. After each meal, for each subject, the amount of food taken (grams), the amount of food eaten (grams) and amount of food eaten (calories) were calculated.

During the first week, the six subjects received placebo for a first half a week (Sunday, Monday, Tuesday) and then a CCK-8 composition for the second half week (Wednesday, Thursday, Friday). Subsequently, the six subjects were invited to participate in an additional two weeks experimental period. During the experimental period, placebo was administered for the entire first week and CCK-8 containing composition was administered for the entire second week. At each meal, the subjects were asked to complete a VAS questionnaire regarding the degree of hunger and fullness before administration, before the meal (5 minutes later) and after the meal. The VAS questionnaire included a 1-10 scale for hunger and fullness, with 3 points of verbal description ("extremely hungry", "not hungry, not full" and "extremely full"). Nausea, discomfort or side effects (verbal description) were recorded by a coordinator.

*Numerical and Statistical Analysis*

The sample size was limited to six subjects. Each subject consumed at least 10 meals with administration of CCK-8 or placebo and the effects on food consumption were compared. Based on prior work with other gastrointestinal tract hormones, a 15% decrease in caloric consumption with a standard deviation of 10% was postulated. The sample size was selected to detect such a difference.

For each subject, data on the amount consumed in each meal was characterized by two parameters meal (B, L, D) and treatment (CCK-8 or placebo). Each meal was treated individually assuming no hierarchy or connection between them and assuming no carry over effect. Sixteen meals were excluded from the final analysis, 11 due to technical problems such as tube insertion or migration, four due to protocol violations such as when a subject had a personal emergency preventing the meal from being finished.

A 3-way unbalanced ANOVA was used to determine if CCK-8 administration had any impact on food consumption was used. The model was formulated as follows:

$$y_{ijkl} = \mu + \alpha_i + \beta_i + \gamma_k + (\alpha\beta)_{ij} + (\beta\gamma)_{jk} + (\alpha\gamma)_{ik} + (\alpha\beta\gamma)_{ijk} + \varepsilon_{ijkl}$$

where $\mu$ is the overall mean, $\alpha_i$ is the effect of the j subject, $\beta_i$ is the treatment effect (CCK-8 or placebo), $\gamma_k$ is the effect of the meal type (B, L, D), $(\alpha\beta)_{ij}$, $(\beta\gamma)_{jk}$, $(\alpha\gamma)_{ik}$ and $(\alpha\beta\gamma)_{ijk}$ are the interaction terms and $\varepsilon_{ijkl}$ is the residual or error term. No period or carry over term was assumed. The *anovan* procedure of MATLAB (The Math Works, Inc.) was used to calculate the ANOVA table where the numbers were not the same numbers of cases for each combination of model and factor (unbalanced).

In addition, daily food consumption for each subject was calculated and expressed as a percentage of consumption reduction. To compare the difference of the mean (and median) both the parametric paired t-test and the non-parametric Wilcoxon rank test were used.

Data were analyzed for each individual and for the group. Analyses were performed for all three measured end points: (1) amount of food consumed (calories), (2) amount of food consumed (grams) and (3) amount taken to plate (grams).

*Results*

The effect of CCK-8 administered in accordance with the teachings of the present invention on the calories consumed is shown in Table III from which is seen that intraduodenal administration of CCK-8 produced a statistically significant reduction in the amount of calories consumed (p=0.0128).

TABLE III: The effect of the CCK-8 administration of the calories consumed

| source | Sum Sq. | d.f. | Mean Sq. | F | Prob > F |
|---|---|---|---|---|---|
| $\alpha_i$ | 1898371.2 | 5 | 379674.2 | 22.01 | 0 |
| $\beta_i$ | 3523282.3 | 2 | 1761641.2 | 102.12 | 0 |
| $\gamma_k$ | 110867 | 1 | 110867 | 6.43 | **0.0128** |
| $(\alpha\beta)_{ij}$ | 727885.5 | 10 | 72788.5 | 4.22 | 0.0001 |
| $(\beta\gamma)_{jk}$ | 134146.3 | 5 | 26829.3 | 1.56 | 0.1799 |
| $(\alpha\gamma)_{ik}$ | 31845.3 | 2 | 15922.7 | 0.92 | 0.4007 |
| $(\alpha\beta\gamma)_{ijk}$ | 112107.7 | 10 | 11210.8 | 0.65 | 0.7676 |
| $\varepsilon_{ijkl}$ | 1690578.2 | 98 | 17250.8 | | |
| Total | 9106845.5 | 133 | | | |

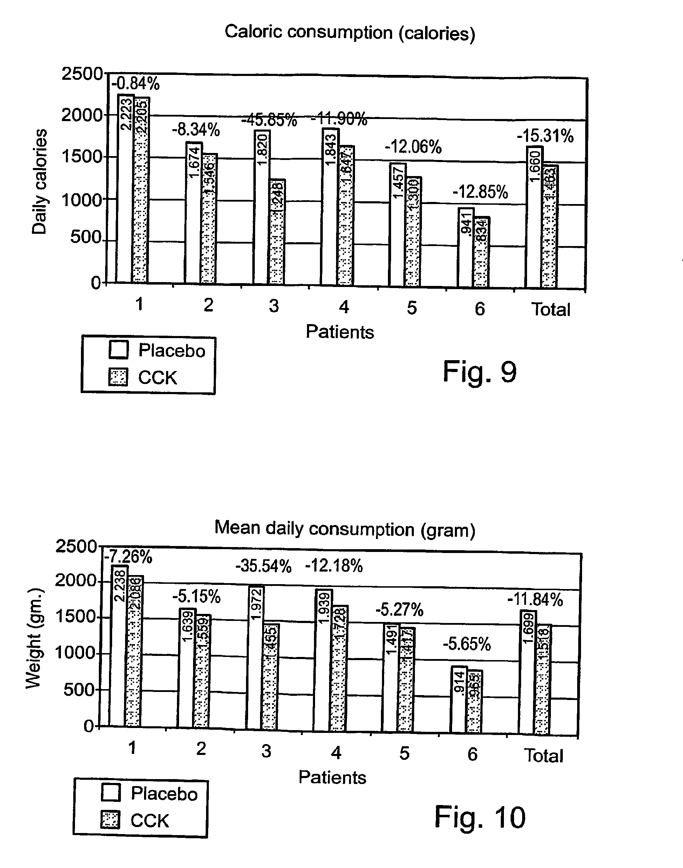

The mean and median amounts of calories consumed by each subject are shown in Figure 9 and in Table IV from which is seen that the mean decrease in food consumption as expressed in calories was $15.31\pm14.25$ % (p=0.03).

TABLE IV: Mean and Median amounts of calories consumed

| | Mean daily consumption (calories) | | Median daily consumption (calories) | |
|---|---|---|---|---|
| Patient | placebo | CCK-8 | placebo | CCK-8 |
| 1 | 2223 | 2205 | 2223 | 2219 |
| 2 | 1674 | 1546 | 1667 | 1546 |
| 3 | 1820 | 1248 | 1765 | 1198 |
| 4 | 1843 | 1647 | 1810 | 1576 |
| 5 | 1457 | 1300 | 1401 | 1331 |
| 6 | 941 | 834 | 878 | 875 |
| Total | 1660 | 1463 | 1624 | 1457 |
| p-value | 0.06 | | 0.12 | |

The effect of CCK-8 administered in accordance with the teachings of the present invention on the meal size consumed is shown in Table V from which is seen that intraduodenal administration of CCK-8 led to a statistically significant reduction in the amount of food consumed (p=0.0164).

TABLE V: The effect of the CCK-8 administration of food consumed in grams

| source | Sum Sq. | d.f. | Mean Sq. | F | Prob > F |
|---|---|---|---|---|---|
| $\alpha_i$ | 1978287.6 | 5 | 395657.5 | 25.24 | 0 |
| $\beta_i$ | 1220448.0 | 2 | 610224.0 | 38.93 | 0 |
| $\gamma_k$ | 93462.4 | 1 | 93462.4 | 5.96 | **0.0164** |

(continued)

| source | Sum Sq. | d.f. | Mean Sq. | F | Prob > F |
|---|---|---|---|---|---|
| $(\alpha\beta)_{ij}$ | 839348.5 | 40 | 83934.9 | 5.36 | 0 |
| $(\beta\gamma)_{jk}$ | 111796.1 | 5 | 22359.2 | 1.43 | 0.2214 |
| $(\alpha\gamma)_{ik}$ | 42419.5 | 2 | 21209.7 | 1.35 | 0.2632 |
| $(\alpha\beta\gamma)_{ijk}$ | 99902.2 | 10 | 9990.2 | 0.64 | 0.7785 |
| $\varepsilon_{ijkl}$ | 1535950.1 | 98 | 15673.0 | | |
| Total | 6105394.9 | 133 | | | |

The effect mean and median amounts of grams of food consumed by each subject are shown in Figure 10 and in Table VI from which is seen that the mean decrease in food consumption as expressed in grams was 11.84±10.87 % (p=0.03).

TABLE VI: Mean and Median amounts of calories consumed

| | Mean daily consumption (grams) | | Median daily consumption (grams) | |
|---|---|---|---|---|
| Patient | placebo | CCK-8 | placebo | CCK-8 |
| 1 | 2238 | 2086 | 2238 | 2134 |
| 2 | 1639 | 1559 | 1659 | 15559 |
| 3 | 1972 | 1455 | 2030 | 1350 |
| 4 | 1939 | 1728 | 2005 | 1763 |
| 5 | 1491 | 1417 | 1495 | 1453 |
| 6 | 914 | 865 | 951 | 866 |
| Total | 1699 | 1518 | 1730 | 1521 |
| p-value | 0.05 | | 0.09 | |

The effect of CCK-8 administered in accordance with the teachings of the present invention on the amount of food taken is shown in Table VII from which is seen that intraduodenal administration of CCK-8 led to a statistically significant reduction in the amount of food consumed (p=0.0219).

TABLE VII: The effect of the CCK-8 administration on food taken in grams

| source | Sum Sq. | d.f. | Mean Sq. | F | Prob > F |
|---|---|---|---|---|---|
| $\alpha_i$ | 2868450.0 | 5 | 573690 | 29.11 | 0 |
| $\beta_i$ | 1940146.9 | 2 | 970073.4 | 49.22 | 0 |
| $\gamma_k$ | 106947 | 1 | 10.6947 | 5.43 | **0.0219** |
| $(\alpha\beta)_{ij}$ | 935578.5 | 10 | 93557.9 | 4.75 | 0 |
| $(\beta\gamma)_{jk}$ | 85368.2 | 5 | 17073.6 | 0.87 | 0.5067 |
| $(\alpha\gamma)_{ik}$ | 54916.6 | 2 | 27458.3 | 1.39 | 0.2531 |
| $(\alpha\beta\gamma)_{ijk}$ | 211799.9 | 10 | 21180 | 1.07 | 0.389 |
| $\varepsilon_{ijkl}$ | 1931379.7 | 98 | 19708.0 | | |
| Total | 8674180.0 | 133 | | | |

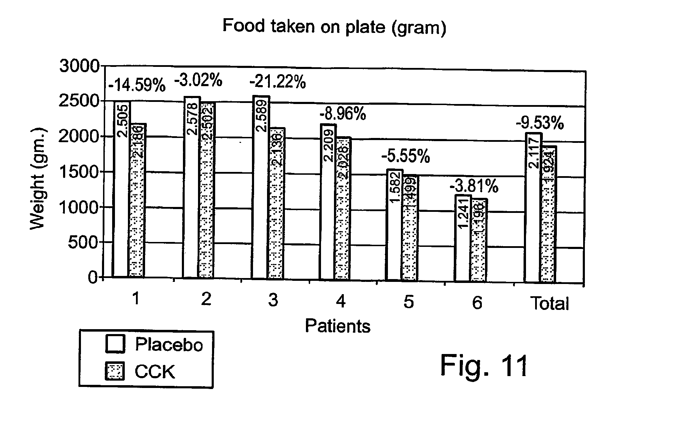

The effect on the mean and median amounts of food taken by each of the six subjects are shown in Figure 11 and in Table VIII from which is seen that the mean decrease in food taken as expressed in grams was 9.53±6.5 % (p=0.03).

TABLE VIII: Mean and Median amounts of food taken (grams)

|  | Mean daily food taken (grams) | | Median daily food taken (grams) | |
| --- | --- | --- | --- | --- |
| Patient | placebo | CCK-8 | placebo | CCK-8 |
| 1 | 2505 | 2186 | 2505 | 2214 |
| 2 | 2578 | 2502 | 2637 | 2502 |
| 3 | 2589 | 2136 | 2645 | 1991 |
| 4 | 2209 | 2028 | 2259 | 2074 |
| 5 | 1582 | 1499 | 1580 | 1522 |
| 6 | 1241 | 1196 | 1198 | 1172 |
| Total | 2117 | 1924 | 2137 | 1912 |
| p-value | 0.03 | | 0.06 | |

*Summary of Results*

As seen from the results presented above and in Figures 9, 10 and 11 and summarized in Table IX, administration of CCK-8 in accordance with the teachings of the present invention led to a significant decrease in the amount of calories consumed, the amount of food consumed and the amount of food taken, even when an unlimited amount of food was available. Administration of CCK-8 in accordance with the teachings of the present invention had no noticeable effect on the VAS scores.

TABLE IX: Summary of influence of CCK-8 administration

| patient | Calories consumed % | Food consumed % | Food taken % |
| --- | --- | --- | --- |
| 1 | 0.83 | 7.26 | 14.59 |
| 2 | 8.31 | 5.15 | 3.02 |
| 3 | 45.83 | 35.54 | 21.22 |
| 4 | 11.90 | 12.18 | 8.96 |
| 5 | 12.06 | 5.27 | 5.55 |
| 6 | 12.83 | 5.65 | 3.81 |
| **Average** | **15.89** | **11.84** | **9.53** |

In conclusion, it was observed that CCK-8 administered in accordance with the teachings of the present invention led to a consistent and stable reduction in caloric consumption, consumption of food (in grams) and amount of food placed on a plate despite the food being freely available in buffet setting. The volunteers did not report any CCK-8 induced nausea, vomiting or abdominal pain. It is concluded that the administration of CCK-8 in accordance with the teachings of the present invention leads to a feeling of satiety. The statistically significant average reduction of about 16% in daily caloric intake corresponds to a potential average weight reduction rate of approximately 1 kg month$^{-1}$. Such a significant weight reduction is associated with improvement in various clinical parameters including reduction of blood glucose levels, reduction of blood pressure and correction of lipid derangements.

Thus, with the study reported above the utility of the gastrointestinal administration of a pharmaceutical composition including a peptide that is a CCK analogue for treating conditions relating to food intake such as obesity, bulimia, eating disorders, overeating, diabetes-related obesity and metabolic syndrome was demonstrated. Thus, the method presented comprises administering a composition that comprises an active agent (e.g., a satiety agent, e.g. CCK or an analogue thereof or a derivative thereof) as an active agent and a pharmaceutically accepted carrier to a subject in need thereof by spraying the composition in a part of the gastrointestinal tract of the subject so that the active agent interacts with the luminal wall of the gastrointestinal tract thereby causing a beneficial effect, for example a reduction of calories consumed and/or a reduction of the amount food consumed. In some embodiments the luminal wall is the luminal wall of the duodenum of the subject where CCKa receptors are located.

The study also teaches the use of satiety agents, such as CCK or an analogue thereof or a derivate thereof (e.g., CCK-8) for the manufacture of a pharmaceutical composition (that is to say, a medicament) for use in the treatment of a subject by gastrointestinal administration. In some embodiments, the treatment comprises reduction of calorie intake by the subject and or reduction of the amount of food consumed by a subject, for example in a subject that suffers from a disorder such as obesity, bulimia, eating disorders, overeating, diabetes-related obesity and metabolic syndrome. In some embodiments, the composition is a sprayable composition. In some embodiments, the gastrointestinal administration is duodenal administration.

More generally, it was demonstrated that gastrointestinal administration of a composition including a peptide active agent for treating conditions is possible. Thus, a method of treatment is presented, comprising administering a composition that comprises a peptide active agent (such as a hormone) and a pharmaceutically accepted carrier to a subject in need thereof by spraying the composition in a part of the gastrointestinal tract of the subject so that the active agent interacts with the luminal wall of the gastrointestinal tract thereby causes a beneficial effect. In some embodiments the luminal wall is the luminal wall of the duodenum of the subject.

The study also teaches the use of peptides as an active agent for the manufacture of a pharmaceutical composition (that is to say, a medicament) for use in the treatment of a subject by gastrointestinal administration. In some embodiments, the composition is a sprayable composition. In some embodiments, the gastrointestinal administration is duodenal administration.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

**Claims**

1. A device for administering a sprayable composition including an active agent to a luminal wall of the gastrointestinal tract of a subject, comprising:

   a) a sprayer configured for deployment in a gastrointestinal tract;
   b) a pressure generator configured to dispense a dose of sprayable composition out through said sprayer as a spray upon actuation; and
   c) an actuator for actuating said pressure generator upon being triggered.

2. The device of claim 1, further comprising:

   d) a composition reservoir functionally associated with said pressure generator so that said pressure generator is configured to force sprayable composition from said reservoir out through said sprayer as a spray upon said actuation.

3. The device of claim 2, further comprising a composition including a peptide active agent and a pharmaceutically acceptable carrier held in said reservoir.

4. The device of claim 3, wherein said active agent is a peptide hormone, an analogue thereof or a derivative thereof.

5. The device of claim 4, wherein said active agent is CCK-8.

6. The device of any of claims 1 to 5, further comprising:

   e) an anchor, configured to anchor said sprayer at a specific location in the gastrointestinal tract.

7. The device of any of claims 1 to 6, further comprising:

   f) an event detector functionally associated with said actuator, configured so that as a result of detection of an event of significance for administration of an active agent, said event detector triggers said actuator.

8. The device of claim 7, wherein said event is a physiological change.

9. The device of any of claims 1 to 8, wherein said sprayer is configured for deployment in the duodenum

10. The device of claim 9, further comprising:

b) a feeder tube including a proximal end functionally associated with said sprayer and a distal end, configured to define a conduit for a sprayable composition from said distal end to said sprayer; and

c) an anchor functionally associated with said distal end of said feeder tube

wherein said anchor and said feeder tube are configured so that when properly deployed in the body of a subject, said feeder tube passes through the pyloric sphincter of said subject to maintain said sprayer in the duodenum of a subject.

11. A sprayable composition comprising a peptide as an active agent for use in the treatment of a subject by gastrointestinal administration.

12. The sprayable composition according to claim 11, wherein said gastrointestinal administration is duodenal administration.

13. The sprayable composition of any of claims 11 or 12, wherein said peptide is a peptide hormone, an analogue thereof or a derivative thereof.

14. The sprayable composition of claim 13, wherein said peptide is CCK-8.

15. The sprayable composition of any of claims 11 to 14, wherein said sprayable composition is configured for treating a condition selected from the group consisting of obesity, bulimia, eating disorders, overeating, diabetes-related obesity, metabolic syndrome, inflammatory bowel disease, infections such as of helicobacter pylori, cardiovascular pathologies such as angina or arrhythmia, asthma and allergies.

**Patentansprüche**

1. Eine Vorrichtung zur Verabreichung einer sprühfähigen Zusammensetzung, einschließlich eines Wirkstoffs, auf eine luminale Wand des Magendarmtrakts einer Person, bestehend aus:

a) einem Zerstäuber zum Einsatz im Magen-Darm-Trakt;
b) einem Druckerzeuger, der dazu dient, um nach erfolgter Aktivierung eine Dosis einer sprühfähigen Zusammensetzung als Spray durch den genannten Zerstäuber abzugeben; und
c) einem Betätiger, der ausgelöst werden kann, um den genannten Druckerzeuger zu aktivieren.

2. Die Vorrichtung nach Anspruch 1, die außerdem Folgendes umfasst:

d) einen Behälter für die Zusammensetzung, der funktionell mit dem genannten Druckerzeuger verbunden ist, welcher so gestaltet ist, dass nach erfolgter Aktivierung des genannten Druckerzeugers aus dem genannten Behälter eine sprühfähige Zusammensetzung als Spray durch den genannten Zerstäuber getrieben wird.

3. Die Vorrichtung nach Anspruch 2, außerdem bestehend aus einem Peptid-Wirkstoff und einer pharmazeutisch akzeptablen Trägersubstanz, welche im Behälter aufbewahrt werden.

4. Die Vorrichtung nach Anspruch 3, worin der genannte Wirkstoff ein Peptidhormon, ein Analogon davon oder ein Derivat davon ist.

5. Die Vorrichtung nach Anspruch 4, worin der genannte Wirkstoff CCK-8 ist.

6. Die Vorrichtung nach einem der Ansprüche 1 bis 5, außerdem bestehend aus:

e) einer Verankerung, die dazu dient, um den genannten Zerstäuber an einer spezifischen Stelle im Gastrointestinaltrakt zu verankern.

7. Die Vorrichtung nach einem der Ansprüche 1 bis 6, außerdem bestehend aus:

f) einem Ereignis-Detektor, der funktionell mit dem genannten Betätiger verbunden ist und so gestaltet ist, dass er den genannten Betätiger auslöst, wenn ein Ereignis, das für die Verabreichung eines Wirkstoffes von Bedeutung ist, entdeckt wurde.

8. Die Vorrichtung nach Anspruch 7, worin das genannte Ereignis eine physiologische Veränderung ist.

9. Die Vorrichtung nach einem der Ansprüche 1 bis 8, worin der genannte Zerstäuber zur Abgabe im Zwölffingerdarm ausgebildet ist.

10. Die Vorrichtung nach Anspruch 9, die überdies Folgendes umfasst:

b) einen Zuleitungsschlauch, der ein proximales Ende, das funktionell mit dem genannten Zerstäuber und einem distalen Ende verbunden ist, beinhaltet und der ausgebildet ist, um eine Leistung für eine sprühfähige Zusammensetzung vom genannten Ende zum genannten Zerstäuber zu definieren: sowie
c) eine Verankerung, die funktionell mit dem genannten distalen Ende des genannten Zuleitungsschlauchs in Verbindung steht, wobei die genannte Verankerung und der genannte Zuleitungsschlauch so gestaltet sind, dass bei sachgemäßer Einbringung in den Körper einer Person der Zuleitungsschlauch durch den Magonpförtnermuskel dieser Person geht, um den genannten Zerstäuber im Zwölffingerdarm der Person zu belassen.

11. Eine sprühfähige Zusammensetzung mit einem Peptid als Wirkstoff zur gastrointestinalen Verabreichung bei der Behandlung einer Person.

12. Die sprühfähige Zusammensetzung nach Anspruch 11, worin es sich bei der genannten gastrointestinalen Verabreichung um eine duodenale Verabreichung handelt.

13. Die sprühfähige Zusammensetzung nach Anspruch 11 oder 12, worin das genannte Peptid ein Peptidhormon, ein Analog davon oder ein Derivat davon ist.

14. Die sprühfähige Zusammensetzung nach Anspruch 13, worin das genannte Peptid CCK-8 ist.

15. Die sprühfähige Zusammensetzung nach einem der Ansprüche 11 bis 14, worin die genannte sprühfähige Zusammensetzung zur Behandlung einer Störung dient, ausgewählt aus der Gruppe bestehend aus: Adipositas, Bulimie, Essstörungen, übermäßiges Essen, mit Diabetes verbundene Adipositas, metabolisches Syndrom, entzündliche Darmerkrankung, Infektionen, etwa mit Helicobacter pylori, Herz-Kreislauf-Erkrankungen wie Angina pectoris oder Herzrhythmusstörungen, Asthma und Allergien.

**Revendications**

1. Dispositif destiné à l'administration d'une composition atomisable renfermant un agent actif sur une paroi luminale du tractus gastro-intestinal d'un sujet, comprenant :

a) un atomiseur conformé pour un déploiement dans un tractus gastro-intestinal ;
b) un générateur de pression conformé pour administrer une dose de composition atomisable par ledit atomiseur lors de son activation ; et
c) un actionneur pour l'activation dudit générateur de pression lors de son déclenchement.

2. Dispositif selon la revendication 1, comprenant en outre:

d) un réservoir de composition fonctionnellement associé audit générateur de pression de manière à ce que ledit générateur de pression soit conformé pour forcer la composition atomisable à s'échapper par l'atomiseur après avoir traversé ledit réservoir lors de ladite activation.

3. Dispositif selon la revendication 2, comprenant en outre une composition renfermant un agent peptide actif et un excipient pharmaceutiquement acceptable contenus dans ledit réservoir.

4. Dispositif selon la revendication 3, dans lequel ledit agent actif est une hormone peptide, un analogue de celle-ci ou un dérivé de celle-ci.

5. Dispositif selon la revendication 4, dans lequel ledit agent actif est du CCK-8.

**6.** Dispositif selon l'une quelconque des revendications 1 à 5, comprenant en outre :

  e) une ancre, conformée pour ancrer ledit atomiseur à un emplacement spécifique dans le tractus gastro-intestinal.

**7.** Dispositif de l'une quelconque des revendications 1 à 6, comprenant en outre :

  f) un détecteur d'événements fonctionnellement associé audit actionneur, conformé de manière à ce qu'en résultat de la détection d'un événement d'importance majeure pour l'administration d'un agent actif, ledit détecteur d'événements déclenche ledit actionneur.

**8.** Dispositif de la revendication 7, dans lequel ledit événement est un changement physiologique.

**9.** Dispositif de l'une quelconque des revendications 1 à 8, dans lequel ledit atomiseur est configuré pour un déploiement dans le duodénum.

**10.** Dispositif de la revendication 9, comprenant en outre :

  b) un tube d'alimentation comprenant une extrémité proximale fonctionnellement associée audit atomiseur et une extrémité distale, conformée pour définir un conduit pour l'écoulement d'une composition atomisable depuis ladite extrémité distale jusqu'audit atomiseur ; et
  c) une ancre fonctionnellement associée à ladite extrémité distale dudit tube d'alimentation, dans lequel ladite ancre et ledit tube d'alimentation sont conformés de manière à ce qu'une fois adéquatement déployés dans le corps d'un sujet, ledit tube d'alimentation passe à travers le sphincter pylorique dudit sujet afin de maintenir ledit atomiseur dans le duodénum d'un sujet.

**11.** Composition atomisable comprenant un peptide à titre d'agent actif destinée à une utilisation dans le traitement d'un sujet par administration gastro-intestinale

**12.** Composition atomisable selon la revendication 11, dans laquelle l'administration gastro-intestinale est une administration duodénale.

**13.** Composition atomisable selon les revendications 11 ou 12, dans laquelle ledit peptide est une hormone peptide, un analogue de celle-ci ou un dérivé de celle-ci.

**14.** Composition atomisable selon la revendication 13, dans laquelle ledit peptide est CCK-8.

**15.** Composition atomisable selon l'une quelconque des revendications 11 à 14, dans laquelle ladite composition atomisable est conformée pour le traitement d'une condition sélectionnée parmi le groupe constitué de : obésité, boulimie, troubles de l'alimentation, suralimentation, obésité liée à un diabète, syndrome métabolique, affection abdominale inflammatoire, infections telles que celles causées par *helicobacter pylori,* pathologies cardiovasculaires telles que l'angine de poitrine ou arythmies, asthme ou allergies.

Fig. 1a

Fig. 1c

Fig. 1b

Fig. 1d

Fig. 2

Fig. 3

Fig. 4a

Fig. 4b

Fig. 4c

94

66

90

84

86

90

92

84

84a

84b

84a

96

84c

84a

Fig. 5b

86

84

82

Fig. 5a

88

84

86

Fig. 6c

Fig. 6b

Fig. 6a

Fig. 6d

Fig. 7

| | Dosage (mcg) | Concentration (mcg/cc) | Contraction (percent) |
|---|---|---|---|
| CCK-clin a | 192 | 30 | 39.02% |
| CCK-barco | 5 | 1.66 | 16.82% |
| CCK-clin a wek 2 | 48 | 8 | 12% |

| | |
|---|---|
| ──o── | CCK-clin a |
| ──□── | CCK-barco |
| ──✳── | CCK-clin a week 2 |

Fig. 8

## Caloric consumption (calories)

**Fig. 9**

Patients — Daily calories

- -0.84% (Patient 1: 2.223 / 2.205)
- -8.34% (Patient 2: 1.674 / 1.546)
- -45.85% (Patient 3: 1.820 / 1.248)
- -11.90% (Patient 4: 1.843 / 1.647)
- -12.06% (Patient 5: 1.457 / 1.300)
- -12.85% (Patient 6: 941 / 834)
- -15.31% (Total: 1.660 / 1.463)

Legend: Placebo / CCK

## Mean daily consumption (gram)

**Fig. 10**

Patients — Weight (gm.)

- -7.26% (Patient 1: 2.238 / 2.086)
- -5.15% (Patient 2: 1.639 / 1.559)
- -35.54% (Patient 3: 1.972 / 1.455)
- -12.18% (Patient 4: 1.939 / 1.728)
- -5.27% (Patient 5: 1.491 / 1.417)
- -5.65% (Patient 6: 914 / 855)
- -11.84% (Total: 1.699 / 1.518)

Legend: Placebo / CCK

Food taken on plate (gram)

Fig. 11

Fig. 12a

Fig. 12c

Fig. 12d

Fig. 12b

Fig. 12e

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IL 20051001053 W
- WO 2006035446 A
- US 6217886 B1
- WO 0134088 A
- US 6472505 B

- WO 2006035446 PCT
- US 20020087113 A
- WO 2006111961 A
- US 20050096637 A
- US 20040220633 A
- US 6154422 A

**Non-patent literature cited in the description**

- **WHITE CL. et al.** *Physiol. Behav.,* 2004, vol. 82 (2-3), 489-96
- **LICHTMAN AH ; CRAVATT BF.** *J. Clin. Invest.,* 2005, vol. 115, 1130-3
- Remington's Pharmaceutical Science
- Remington's Pharmaceutical Sciences. Mack Publishing Co,
- **C.A. RAMSDEN GD.** Quantitative Drug Design. Pergamon Press, 1992
- **J. M. STEWART ; J. D. YOUNG.** Solid Phase Peptide Synthesis. W. H. Freeman Co, 1963
- **J. MEIENHOFER.** Hormonal Proteins and Peptides. Academic Press, 1973, vol. 2, 46
- **G. SCHRODER ; K. LUPKE.** The Peptides. Academic Press, 1965, vol. 1

- **ANDERSSON.** *Biopolymers,* 2000, vol. 55 (3), 227-50
- **LE ROUX CW. et al.** *Endocrinology,* 15 September 2005
- **STANLEY S. et al.** *Am. J. Physiol. Gastrointest. Liver Physiol.,* 2004, vol. 286 (5), G693
- **QIN X ; TSO P.** *Curr Drug Targets.,* 2005, vol. 6 (2), 145-51
- **KOBELT P.** *Gut.,* 30 June 2005
- **MISKOWIAK J et al.** *Regul. Pept.,* 1985, vol. 12, 231-6
- **ARCHER-LAHLOU E et al.** *J. Biol. Chem.,* 2005, vol. 280, 10664-10674